# EUROPEAN PATENT APPLICATION

(11) **EP 2 474 542 A1**
(43) Date of publication of application: **11.07.2012**
(21) Application number: 10356034.8
(22) Date of filing: 29.12.2010
(51) Int. Cl.: C07D 417/12, A01N 43/713

(54) **Fungicide hydroximoyl-tetrazole derivatives**

(71) Applicant: Bayer CropScience AG, 40789 Monheim (DE)
(72) Inventor: The designation of the inventor has not yet been filed
(74) Representative: Guitton, Carole

(57) **Abstract**

The present invention relates to hydroximoyl-heterocycle derivatives of formula (I) 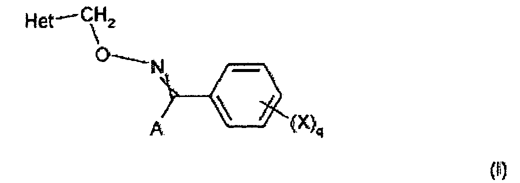 wherein X represents various substituents, A represents a tetrazoyl group, and Het represents a thiazolyl group of formula:

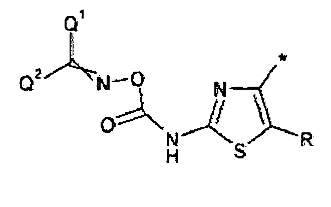

## Description

The present invention relates to hydroximoyl-tetrazole derivatives, their process of preparation, their use as fungicide active agents, particularly in the form of fungicide compositions and methods for the control of phytopathogenic fungi, notably of plants, using these compounds or compositions.

In European patent application n°1426371, there are disclosed certain tetrazoyloxime derivatives of the following chemical structure: 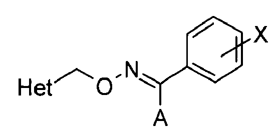 wherein A represents a tetrazolyl group, Het represents either a particular pyridinyl group or a particular thiazolyl group.

In Japanese patent application n°2004-131392, there are disclosed certain tetrazoyloxime derivatives of the following chemical structure: 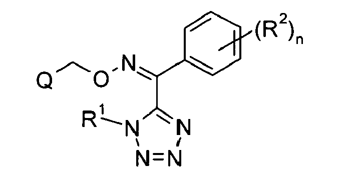 wherein Q can be selected in a list of 15 various heterocycle groups.

In Japanese patent application n°2004-131416, there are disclosed certain tetrazoyloxime derivatives of the following chemical structure: 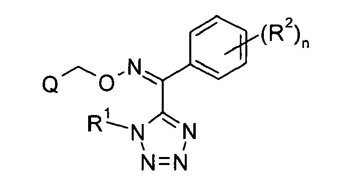 wherein Q can be selected among a pyridinyl group or a thiazolyl group.

The compounds disclosed in these three documents do not prove to provide a comparable utility than the compounds according to the invention.

It is always of high-interest in agriculture to use novel pesticide compounds in order to avoid or to control the development of resistant strains to the active ingredients. It is also of high-interest to use novel compounds being more active than those already known, with the aim of decreasing the amounts of active compound to be used, whilst at the same time maintaining effectiveness at least equivalent to the already known compounds. We have now found a new family of compounds which possess the above mentioned effects or advantages.

Accordingly, the present invention provides a tetrazoyloxime derivative of formula (I) 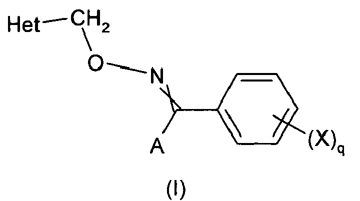 wherein
• X independently represents a hydrogen atom, a halogen atom, a nitro group, a hydroxy group, a cyano group, hydroxycarbonyl, C₁-C₈-alkoxycarbonyl, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶-sulphenyl group, a formylamino group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl-C₁-C₈-alkyl, substituted or non-substituted C₃-C₈-cycloalkyl, substituted or non-substituted tri(C₁-C₈-alkyl)silyl-C₃-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted N-(C₁-C₈-alkoxy)-C₁-C₈-alkanimidoyl, substituted or non-substituted N-(C₁-C₈-alkoxy)-C₁-C₈-halogenoalkanimidoyl having 1 to 5 halogen atoms, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms , substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted tri(C₁-C₈-alkyl)-silyl, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy, substituted or non-substituted [(arylcarbonyl)amino]-[C₁-C₈]-alkyl, substituted or non-substituted [{C₁-C₈-alkyl(C₁-C₈-alkylcarbonyl)amino}]-[C₁-C₈]-alkyl, substituted or non-substituted [{C₁-C₈-alkyl(arylcarbonyl)amino}]-[C₁-C₈]-alkyl, substituted or non-substituted [(C₁-C₈-alkylcarbonyl)amino]-[C₁-C₈]-alkyl , substituted or non-substituted heterocyclyl, substituted or non-substituted heterocyclyloxy;
• q represents 1, 2, 3, 4 or 5;
• A represents a tetrazoyl group of formula (A¹) or (A²): 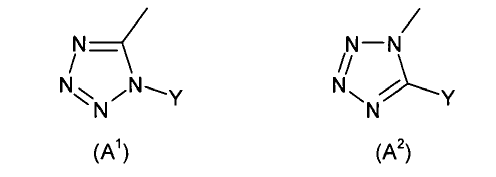 wherein Y represents substituted or non-substituted C₁-C₈-alkyl; and
• Het represents a thiazolyl group of formula: 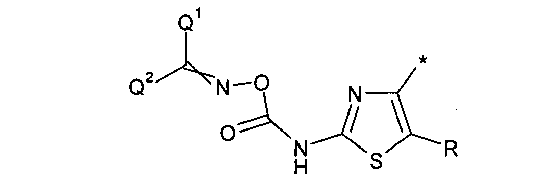 wherein
   o R represents a hydrogen atom, a halogen atom, C₁-C₈-alkyl or C₁-C₈-alkoxy and
   o Q¹ represents a hydrogen atom, a hydroxy group, a cyano group, substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted C₁-C₈-alkoxy, an amino group, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S; substituted or non-substituted C₁-C₆-alkyl-(hydroxyimino)-, substituted or non-substituted C₁-C₆-alkyl-(C₁-C₆-alkoxyimino)-, substituted or non-substituted C₁-C₆-alkyl-(C₂-C₆-alkenyloxyimino)-, substituted or non-substituted C₁-C₆-alkyl-(C₂-C₆-alkynyloxyimino)-, substituted or non-substituted C₁-C₆-alkyl-(benzyloxyimino)-, substituted or non-substituted heterocyclyl-(C₁-C₆-alkoxyimino)-, substituted or non-substituted heterocyclyl-(C₂-C₆-alkenyloxyimino)-, substituted or non-substituted heterocyclyl-(C₂-C₆-alkynyloxyimino)-, substituted or non-substituted heterocyclyl-(benzyloxyimino)-, substituted or non-substituted aryl-(C₁-C₆-alkoxyimino)-, substituted or non-substituted aryl-(C₂-C₆-alkenyloxyimino)-, substituted or non-substituted aryl-(C₂-C₆-alkynyloxyimino)-, substituted or non-substituted aryl-(benzyloxyimino)-, substituted or non-substituted C₅-C₁₂-fused bicycloalkyl, substituted or non-substituted C₅-C₁₂-fused bicycloalkenyl, substituted or non-substituted C₁-C₆-allenyl;
   o Q² represents a substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted C₁-C₈-alkoxy, substituted or non-substituted aryl, substituted or non-substituted, saturated or unsaturated 4-, 5-, 6-, 7-, 8-, 9- , 10-, or 11-membered heterocyclyl comprising up to 4 heteroatoms selected in the list consisting of N, O, S; substituted or non-substituted C₁-C₆-alkyl-(hydroxyimino)-, substituted or non-substituted C₁-C₆-alkyl-(C₁-C₆-alkoxyimino)-, substituted or non-substituted C₁-C₆-alkyl-(C₂-C₆-alkenyloxyimino)-, substituted or non-substituted C₁-C₆-alkyl-(C₂-C₆-alkynyloxyimino)-, substituted or non-substituted C₁-C₆-alkyl-(benzyloxyimino)-, substituted or non-substituted heterocyclyl-(C₁-C₆-alkoxyimino)-, substituted or non-substituted heterocyclyl-(C₂-C₆-alkenyloxyimino)-, substituted or non-substituted heterocyclyl-(C₂-C₆-alkynyloxyimino)-, substituted or non-substituted heterocyclyl-(benzyloxyimino)-, substituted or non-substituted aryl-(C₁-C₆-alkoxyimino)-, substituted or non-substituted aryl-(C₂-C₆-alkenyloxyimino)-, substituted or non-substituted aryl-(C₂-C₆-alkynyloxyimino)-, substituted or non-substituted aryl-(benzyloxyimino)-, substituted or non-substituted C₅-C₁₂-fused bicycloalkyl, substituted or non-substituted C₅-C₁₂-fused bicycloalkenyl, substituted or non-substituted C₁-C₆-allenyl; or
   o Q¹ and Q² form together a substituted or non-substituted, saturated or unsaturated 4-, 5-, 6-, 7-, 8-, 9-, 10-, or 11-membered carbocycle or heterocycle comprising up to 4 heteroatoms selected in the list consisting of N, O, S;
   as well as salts, N-oxides, metallic complexes and metalloidic complexes thereof or (E) and (Z) isomers and mixtures thereof.

Any of the compounds according to the invention can exist as one or more stereoisomers depending on the number of stereogenic units (as defined by the IUPAC rules) in the compound. The invention thus relates equally to all the stereoisomers, and to the mixtures of all the possible stereoisomers, in all proportions. The stereoisomers can be separated according to the methods which are known per se by the man ordinary skilled in the art.
Notably, the stereostructure of the oxime moieties present in the tetrazolyloxime derivative of formula (I) includes (E) or (Z) isomer, and these stereoisomers form part of the present invention.

According to the invention, the following generic terms are generally used with the following meanings:
- halogen means fluorine, chlorine, bromine or iodine ;
- heteroatom can be nitrogen, oxygen or sulphur ;
- unless indicated otherwise, a group or a substituent that is substituted according to the invention can be substituted by one or more of the following groups or atoms: a halogen atom, a nitro group, a hydroxy group, a cyano group, an amino group, a sulphenyl group, a pentafluoro-λ⁶-sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a formylamino group, a (hydroxyimino)-C₁-C₆-alkyl group, a C₁-C₈-alkyl, a tri(C₁-C₈-alkyl)silyl-C₁-C₈-alkyl, C₁-C₈-cycloalkyl, tri(C₁-C₈-alkyl)silyl-C₁-C₈-cycloalkyl, a C₁-C₈-halogenoalkyl having 1 to 5 halogen atoms, a C₁-C₈-halogenocycloalkyl having 1 to 5 halogen atoms, a C₂-C₈-alkenyl, a C₂-C₈-alkynyl, a C₂-C_{B}-alkenyloxy, a C₂-C₈-alkynyloxy, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulphenyl, a C₁-C₈-halogenoalkylsulphenyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, substituted or non-substituted C₁-C₈-alkoxycarbonylamino, substituted or non-substituted C₁-C₈-halogenoalkoxycarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₈-alkylaminosulfamoyl, a di-C₁-C₈-alkylaminosulfamoyl, a (C₁-C₆-alkoxyimino)-C₁-C₆-alkyl, a (C₁-C₆-alkenyloxyimino)-C₁-C₆-alkyl, a (C₁-C₆-alkynyloxyimino)-C₁-C₆-alkyl, (benzyloxyimino)-C₁-C₆-alkyl, C₁-C₈-alkoxyalkyl, C₁-C₈-halogenoalkoxyalkyl having 1 to 5 halogen atoms, benzyloxy, benzylsulphenyl, benzylamino, phenoxy, phenylsulphenyl, or phenylamino ;

- the term "aryl" means phenyl or naphthyl;
- the term "heterocyclyl" means saturated or unsaturated 4-, 5-, 6- or 7-membered ring comprising up to 4 heteroatoms selected in the list consisting of N, O, S.

Preferred compounds of formula (I) according to the invention are those wherein the substitution position of X is not specifically limited.
Other preferred compounds of formula (I) according to the invention are those wherein X represents a hydrogen atom, a halogen atom, substituted or non-substituted C₁-C₈-alkyl, a substituted or non-substituted C₁-C₈-alkoxy, a cyano group, a methanesulfonyl group, a nitro group, a trifluoromethyl group or an aryl group.
Amongst the halogen atoms, a chlorine atom or a fluorine atom is particularly preferred.
The substituted or non-substituted C₁-C₈-alkyl group represented for X is preferably an alkyl group having 1 to 4 carbon atoms and specific examples thereof include a methyl group, an ethyl group, an n-propyl group, an isopropyl group, an n-butyl group, an isobutyl group, a sec-butyl group, and a tert-butyl group. Among these alkyl groups, a methyl group or a tert-butyl group is particularly preferred.
The alkoxy group for X is preferably a substituted or non-substituted C₁-C₈-alkoxy group having 1 to 3 carbon atoms and specific examples thereof include a methoxy group, an ethoxy group, a propoxy group, and an isopropoxy group. Among these alkoxy groups, a methoxy group or an ethoxy group is particularly preferred.

Even more preferred compounds of formula (I) according to the invention are those wherein X represents a hydrogen atom.
Preferred compounds of formula (I) according to the invention are those wherein q represents 1

Other preferred compounds of formula (I) according to the invention are those wherein Y represents a substituted or non-substituted C₁-C₈-alkyl group. Among these alkyl groups, an alkyl group having 1 to 3 carbon atoms such as a methyl group, an ethyl group, an n-propyl group or an isopropyl group is preferable. Among these alkyl groups, a methyl group or an ethyl group is particularly preferred.

Other preferred compounds of formula (I) according to the invention are those wherein R represents a hydrogen atom or a halogen atom such as a chlorine atom, a bromine atom, an iodine atom or a fluorine atom. Among these, a hydrogen atom or a chlorine atom is particularly preferred.

Other preferred compounds of formula (I) according to the invention are those wherein Q¹ represents a hydrogen atom, substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S.
More preferred compounds of formula (I) according to the invention are those wherein Q¹ represents a hydrogen atom, substituted or non-substituted C₁-C₈-alkyl.

Other preferred compounds of formula (I) according to the invention are those wherein Q² represents a substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S.

Other preferred compounds of formula (I) according to the invention are those wherein Q¹ and Q² form together a substituted or non-substituted, saturated or unsaturated 5-, 6-, 7-, 8-, 9-, 10-, or 11-membered carbocycle or heterocycle comprising up to 4 heteroatoms selected in the list consisting of N, O, S.
More preferred compounds of formula (I) according to the invention are those wherein Q¹ and Q² form together a substituted or non-substituted cyclopentyl or cyclohexyl.

The above mentioned preferences with regard to the substituents of the compounds of formula (I) according to the invention can be combined in various manners. These combinations of preferred features thus provide sub-classes of compounds according to the invention. Examples of such subclasses of preferred compounds according to the invention can combine:
- preferred features of X with preferred features of one or more of A, Y, Het, q, R, Q₁ and Q₂ ;
- preferred features of A with preferred features of one or more of X, Y, Het, q, R, Q₁ and Q₂ ;
- preferred features of Y with preferred features of one or more of A, X, Het, q, R, Q₁ and Q₂ ;
- preferred features of Het with preferred features of one or more of A, Y, X, q, R, Q₁ and Q₂ ;
- preferred features of q with preferred features of one or more of A, Y, Het, X, R, Q₁ and Q₂ ;
- preferred features of R with preferred features of one or more of A, Y, Het, q, X, Q₁ and Q₂
- preferred features of Q₁ with preferred features of one or more of A, Y, Het, q, R, X and Q₂ ;
- preferred features of Q₂ with preferred features of one or more of A, Y, Het, q, R, Q₁ and X .

In these combinations of preferred features of the substituents of the compounds according to the invention, the said preferred features can also be selected among the more preferred features of each of X, A, Y, Het, q, R, Q₁ and Q₂ ; so as to form most preferred subclasses of compounds according to the invention.

The present invention also relates to a process for the preparation of compounds of formula (I). Thus, according to a further aspect of the present invention, there is a provided process P1 for the preparation of compounds of formula (I), as herein-defined, as illustrated by the following reaction scheme: 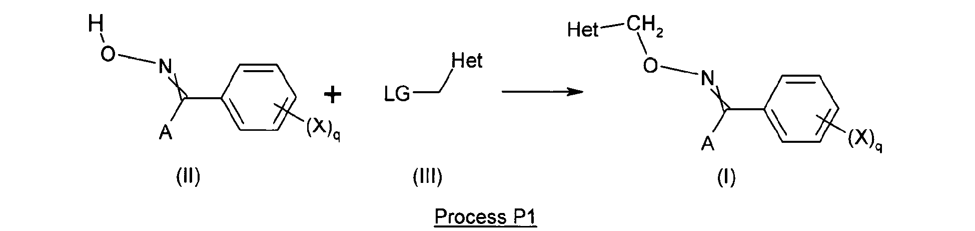 wherein A, X, Z, q and Het are as herein-defined and LG represents a leaving group. Suitable leaving groups can be selected in the list consisting of a halogen atom or other customary nucleofugal groups such as triflate, mesylate or tosylate.

For the compounds of formula (Ia), process P1 according to the invention can be completed by a further step comprising the additional modification of this group, notably by a reaction of acylation to yield to a compound of formula (Ib), according to known methods. In such a case there is provided a process P2 according to the invention and such a process P2 can be illustrated by the following reaction scheme : 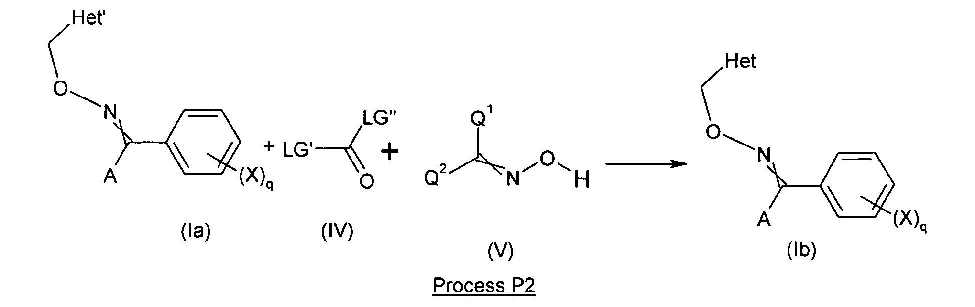
• wherein A, X, q, Q¹, Q² and Het are as herein-defined, LG' and LG" independently represent a leaving group, Het' represents a thiazolyl group of formula (Het'¹) ;

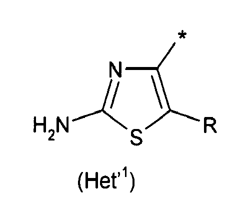

Suitable leaving groups can be selected in the list consisting of a halogen atom or other customary nucleofugal groups such as alcoholate, halogenoalcoholate or substititued phenolate.
For the compounds of formula (Ia), carrying out process P2 would previously require a deprotection step in order to yield the amino group. Amino-protecting groups and related methods of cleavage thereof are known to the ordinary skilled man in the art.

According to the invention, processes P1 and P2 can be performed if appropriate in the presence of a solvent and if appropriate in the presence of a base.

According to the invention, processes P1 and P2 can be performed if appropriate in the presence of a catalyst. Suitable catalyst can be selected in the list consisting of 4-dimethyl-aminopyridine, 1-hydroxybenzotriazole or dimethylformamide.

Suitable solvents for carrying out processes P1 and P2 according to the invention are customary inert organic solvents. Preference is given to using optionally halogenated aliphatic, alicyclic or aromatic hydrocarbons, such as petroleum ether, hexane, heptane, cyclohexane, methylcyclohexane, benzene, toluene, xylene or decalin ; chlorobenzene, dichlorobenzene, dichloromethane, chloroform, carbon tetrachloride, dichlorethane or trichlorethane ; ethers, such as diethyl ether, diisopropyl ether, methyl tert-butyl ether, methyl tert-amyl ether, dioxane, tetrahydrofuran, 1,2-dimethoxyethane, 1,2-diethoxyethane or anisole ; nitriles, such as acetonitrile, propionitrile, n- or iso-butyronitrile or benzonitrile ; amides, such as N,N-dimethylformamide, N,N-dimethylacetamide, N-methylformanilide, N-methylpyrrolidone or hexamethylphosphoric triamide ; esters, such as methyl acetate or ethyl acetate, sulphoxides, such as dimethyl sulphoxide, or sulphones, such as sulpholane.

Suitable bases for carrying out processes P1 and P2 according to the invention are inorganic and organic bases which are customary for such reactions. Preference is given to using alkaline earth metal, alkali metal hydride, alkali metal hydroxides or alkali metal alkoxides, such as sodium hydroxide, sodium hydride, calcium hydroxide, potassium hydroxide, potassium tert-butoxide or other ammonium hydroxide, alkali metal carbonates, such as sodium carbonate, potassium carbonate, potassium bicarbonate, sodium bicarbonate, cesium carbonate, alkali metal or alkaline earth metal acetates, such as sodium acetate, potassium acetate, calcium acetate, and also tertiary amines, such as trimethylamine, triethylamine, diisopropylethylamine, tributylamine, N,N-dimethylaniline, pyridine, N-methylpiperidine, N,N-dimethyl-aminopyridine, 1,4-diazabicyclo[2.2.2]octane (DABCO), 1,5-diazabicyclo[4.3.0]non-5-ene (DBN) or 1,8-diazabicyclo[5.4.0]undec-7-ene (DBU).

When carrying out processes P1 and P2 according to the invention, the reaction temperature can independently be varied within a relatively wide range.
Generally, process P1 according to the invention is carried out at temperatures between -20°C and 160°C.

Processes P1 and P2 according to the invention are generally independently carried out under atmospheric pressure. However, it is also possible to operate under elevated or reduced pressure.

When carrying out process P1 according to the invention, generally 1 mol or an excess of derivative of formula Het-CH₂-LG and from 1 to 3 mol of base are employed per mole of hydroximoyl tetrazole of formula (II). It is also possible to employ the reaction components in other ratios.

Work-up is carried out by customary methods. Generally, the reaction mixture is treated with water and the organic phase is separated off and, after drying, concentrated under reduced pressure. If appropriate, the remaining residue can be freed by customary methods, such as chromatography or recrystallization, from any impurities that can still be present.

Compounds according to the invention can be prepared according to the above described processes. It will nevertheless be understood that, on the basis of his general knowledge and of available publications, the skilled worker will be able to adapt these processes according to the specifics of each of the compounds according to the invention that is desired to be synthesised.

When A represents a substituent of formula A¹, as herein-described, the compounds of formula (II), useful as a starting material, can be prepared, for example, by reacting hydroxylamine with the corresponding ketones that can be prepared, for example, according to the method described by R. Raap (*Can. J. Chem.* 1971, 49, 2139) by addition of a tetrazolyl lithium species to esters of formula 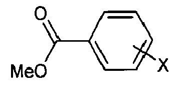 or 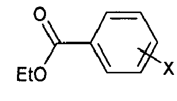 or any of their suitable synthetic equivalents like, for example : 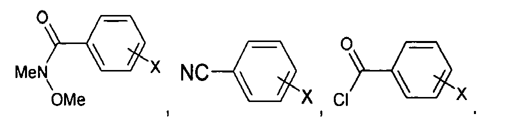 When A represents a substituent of formula A², as herein-described, the compounds of general formula (II) useful as a starting material, can be prepared, for example, from oximes of formula 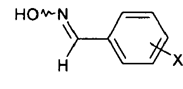 and 5-substituted tetrazole according to the method described by J. Plenkiewicz et al. (*Bull. Soc. Chim. Belg.* 1987, 96, 675).

In a further aspect, the present invention also relates to a fungicide composition comprising an effective and non-phytotoxic amount of an active compound of formula (I).

The expression "effective and non-phytotoxic amount" means an amount of composition according to the invention which is sufficient to control or destroy the fungi present or liable to appear on the crops and which does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials, which are within the capabilities of a person skilled in the art.

Thus, according to the invention, there is provided a fungicide composition comprising, as an active ingredient, an effective amount of a compound of formula (I) as herein defined and an agriculturally acceptable support, carrier or filler.

According to the invention, the term "support" denotes a natural or synthetic organic or inorganic compound with which the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol organic solvents, mineral and plant oils and derivatives thereof. Mixtures of such supports can also be used.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols and derivatives of the above compounds containing sulphate, sulphonate and phosphate functions. The presence of at least one surfactant is generally essential if the active compound and/or the inert support are water-insoluble and if the vector agent for the application is water. Preferably, surfactant content can be comprised from 5% to 40% by weight of the composition.

Optionally, additional components can also be included, e.g. protective colloids, adhesives, thickeners, thixotropic agents, penetration agents, stabilisers, sequestering agents. More generally, the active compounds can be combined with any solid or liquid additive, which complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions which are ready to be applied to the plant or seed to be treated by means of a suitable device, such as a spraying or dusting device, but also concentrated commercial compositions which must be diluted before application to the crop.

The compounds according to the invention can also be mixed with one or more insecticide, fungicide, bactericide, attractant, acaricide or pheromone active substance or other compounds with biological activity. The mixtures thus obtained have a broadened spectrum of activity. The mixtures with other fungicide compounds are particularly advantageous. The composition according to the invention comprising a mixture of a compound of formula (I) with a bactericide compound can also be particularly advantageous.

Examples of suitable fungicide mixing partners can be selected in the following lists:
(1) Inhibitors of the ergosterol biosynthesis, for example (1.1) aldimorph (1704-28-5), (1.2) azaconazole (60207-31-0), (1.3) bitertanol (55179-31-2), (1.4) bromuconazole (116255-48-2), (1.5) cyproconazole (113096-99-4), (1.6) diclobutrazole (75736-33-3), (1.7) difenoconazole (119446-68-3), (1.8) diniconazole (83657-24-3), (1.9) diniconazole-M (83657-18-5), (1.10) dodemorph (1593-77-7), (1.11) dodemorph acetate (31717-87-0), (1.12) epoxiconazole (106325-08-0), (1.13) etaconazole (60207-93-4), (1.14) fenarimol (60168-88-9), (1.15) fenbuconazole (114369-43-6), (1.16) fenhexamid (126833-17-8), (1.17) fenpropidin (67306-00-7), (1.18) fenpropimorph (67306-03-0), (1.19) fluquinconazole (136426-54-5), (1.20) flurprimidol (56425-91-3), (1.21) flusilazole (85509-19-9), (1.22) flutriafol (76674-21-0), (1.23) furconazole (112839-33-5), (1.24) furconazole-cis (112839-32-4), (1.25) hexaconazole (79983-71-4), (1.26) imazalil (60534-80-7), (1.27) imazalil sulfate (58594-72-2), (1.28) imibenconazole (86598-92-7), (1.29) ipconazole (125225-28-7), (1.30) metconazole (125116-23-6), (1.31) myclobutanil (88671-89-0), (1.32) naftifine (65472-88-0), (1.33) nuarimol (63284-71-9), (1.34) oxpoconazole (174212-12-5), (1.35) paclobutrazol (76738-62-0), (1.36) pefurazoate (101903-30-4), (1.37) penconazole (66246-88-6), (1.38) piperalin (3478-94-2), (1.39) prochloraz (67747-09-5), (1.40) propiconazole (60207-90-1), (1.41) prothioconazole (178928-70-6), (1.42) pyributicarb (88678-67-5), (1.43) pyrifenox (88283-41-4), (1.44) quinconazole (103970-75-8), (1.45) simeconazole (149508-90-7), (1.46) spiroxamine (118134-30-8), (1.47) tebuconazole (107534-96-3), (1.48) terbinafine (91161-71-6), (1.49) tetraconazole (112281-77-3), (1.50) triadimefon (43121-43-3), (1.51) triadimenol (89482-17-7), (1.52) tridemorph (81412-43-3), (1.53) triflumizole (68694-11-1), (1.54) triforine (26644-46-2), (1.55) triticonazole (131983-72-7), (1.56) uniconazole (83657-22-1), (1.57) uniconazole-p (83657-17-4), (1.58) viniconazole (77174-66-4), (1.59) voriconazole (137234-62-9), (1.60) 1-(4-chlorophenyl)-2-(1H-1,2,4-triazol-1-yl)cycloheptanol (129586-32-9), (1.61) methyl 1-(2,2-dimethyl-2,3-dihydro-1H-inden-1-yl)-1H-imidazole-5-carboxylate (110323-95-0), (1.62) N'-{5-(difluoromethyl)-2-methyl-4-[3-(trimethylsilyl)propoxy]phenyl}-N-ethyl-N-methylimidoformamide, (1.63) N-ethyl-N-methyl-N'-{2-methyl-5-(trifluoromethyl)4-[3-(trimethylsilyl)propoxy]phenyl}imidoformamide and (1.64) O-[1-(4-methoxyphenoxy)-3,3-dimethylbutan-2-yl] 1 H-imidazole-1-carbothioate (111226-71-2).
(2) inhibitors of the respiratory chain at complex I or II, for example (2.1) bixafen (581809-46-3), (2.2) boscalid (188425-85-6), (2.3) carboxin (5234-68-4), (2.4) diflumetorim (130339-07-0), (2.5) fenfuram (24691-80-3), (2.6) fluopyram (658066-35-4), (2.7) flutolanil (66332-96-5), (2.8) fluxapyroxad (907204-31-3), (2.9) furametpyr (123572-88-3), (2.10) furmecyclox (60568-05-0), (2.11) isopyrazam (mixture of syn-epimeric racemate 1RS,4SR,9RS and anti-epimeric racemate 1 RS,4SR,9SR) (881685-58-1), (2.12) isopyrazam (anti-epimeric racemate 1RS,4SR,9SR), (2.13) isopyrazam (anti-epimeric enantiomer 1R,4S,9S), (2.14) isopyrazam (anti-epimeric enantiomer 1S,4R,9R), (2.15) isopyrazam (syn epimeric racemate 1 RS,4SR,9RS), (2.16) isopyrazam (syn-epimeric enantiomer 1R,4S,9R), (2.17) isopyrazam (syn-epimeric enantiomer 1S,4R,9S), (2.18) mepronil (55814-41-0), (2.19) oxycarboxin (5259-88-1), (2.20) penflufen (494793-67-8), (2.21) penthiopyrad (183675-82-3), (2.22) sedaxane (874967-67-6), (2.23) thifluzamide (130000-40-7), (2.24) 1-methyl-N-[2-(1,1,2,2-tetrafluoroethoxy)phenyl]-3-(trifluoromethyl)-1H-pyrazole-4-carboxamide, (2.25) 3-(difluoromethyl)-1-methyl-N-[2-(1,1,2,2-tetrafluoroethoxy)phenyl]-1H-pyrazole-4-carboxamide, (2.26) 3-(difluoromethyl)-N-[4-fluoro-2-(1,1,2,3,3,3-hexafluoropropoxy)phenyl]-1-methyl-1H-pyrazole-4-carboxamide, (2.27) N-[1-(2,4-dichlorophenyl)-1-methoxypropan-2-yl]-3-(difluoromethyl)-1-methyl-1H-pyrazole-4-carboxamide (1092400-95-7) (WO 2008148570), (2.28) 1-methyl-3-(trifluoromethyl)-N-[2'-(trifluoromethyl)biphenyl-2-yl]-1H-pyrazole-4-carboxamide, (2.29) N-(4'-chlorobiphenyl-2-yl)-3-(difluoromethyl)-1-methyl-1H-pyrazole-4-carboxamide, (2.30) N-(2',4'-dichlorobiphenyl-2-yl)-3-(difluoromethyl)-1-methyl-1H-pyrazole-4-carboxamide, (2.31) 3-(difluoromethyl)-1-methyl-N-[4'-(trifluoromethyl)biphenyl-2-yl]-1H-pyrazole-4-carboxamide, (2.32) N-(2',5'-difluorobiphenyl-2-yl)-1-methyl-3-(trifluoromethyl)-1H-pyrazole-4-carboxamide, (2.33) 3-(difluoromethyl)-1-methyl-N-[4'-(prop-1-yn-1-yl)biphenyl-2-yl]-1H-pyrazole-4-carboxamide (known from WO 2004/058723), (2.34) 5-fluoro-1,3-dimethyl-N-[4'-(prop-1-yn-1-yl)biphenyl-2-yl]-1H-pyrazole-4-carboxamide (known from WO 2004/058723), (2.35) 2-chloro-N-[4'-(prop-1-yn-1-yl)biphenyl-2-yl]pyridine-3-carboxamide (known from WO 2004/058723), (2.36) 3-(difluoromethyl)-N-[4'-(3,3-dimethylbut-1-yn-1-yl)biphenyl-2-yl]-1-methyl-1 H-pyrazole-4-carboxamide (known from WO 2004/058723), (2.37) N-[4'-(3,3-dimethylbut-1-yn-1-yl)biphenyl-2-yl]-5-fluoro-1,3-dimethyl-1H-pyrazole-4-carboxamide (known from WO 2004/058723), (2.38) 3-(difluoromethyl)-N-(4'-ethynylbiphenyl-2-yl)-1-methyl-1H-pyrazole-4-carboxamide (known from WO 2004/058723), (2.39) N-(4'-ethynylbiphenyl-2-yl)-5-fluoro-1,3-dimethyl-1H-pyrazole-4-carboxamide (known from WO 2004/058723), (2.40) 2-chloro-N-(4'-ethynylbiphenyl-2-yl)pyridine-3-carboxamide (known from WO 2004/058723), (2.41) 2-chloro-N-[4'-(3,3-dimethylbut-1-yn-1-yl)biphenyl-2-yl]pyridine-3-carboxamide (known from WO 2004/058723), (2.42) 4-(difluoromethyl)-2-methyl-N-[4'-(trifluoromethyl)biphenyl-2-yl]-1,3-thiazole-5-carboxamide (known from WO 2004/058723), (2.43) 5-fluoro-N-[4'-(3-hydroxy-3-methylbut-1-yn-1-yl)biphenyl-2-yl]-1,3-dimethyl-1H-pyrazole-4-carboxamide (known from WO 2004/058723), (2.44) 2-chloro-N-[4'-(3-hydroxy-3-methylbut-1-yn-1-yl)biphenyl-2-yl]pyridine-3-carboxamide (known from WO 2004/058723), (2.45) 3-(difluoromethyl)-N-[4'-(3-methoxy-3-methylbut-1-yn-1-yl)biphenyl-2-yl]-1-methyl-1H-pyrazole-4-carboxamide (known from WO 2004/058723), (2.46) 5-fluoro-N-[4'-(3-methoxy-3-methylbut-1-yn-1-yl)biphenyl-2-yl]-1,3-dimethyl-1H-pyrazole-4-carboxamide (known from WO 2004/058723), (2.47) 2-chloro-N-[4'-(3-methoxy-3-methylbut-1-yn-1-yl)biphenyl-2-yl]pyridine-3-carboxamide (known from WO 2004/058723) and salts thereof.
(3) inhibitors of the respiratory chain at complex III, for example (3.1) ametoctradin (865318-97-4), (3.2) amisulbrom (348635-87-0), (3.3) azoxystrobin (131860-33-8), (3.4) cyazofamid (120116-88-3), (3.5) dimoxystrobin (141600-52-4), (3.6) enestroburin (238410-11-2) (WO 2004/058723), (3.7) famoxadone (131807-57-3) (WO 2004/058723), (3.8) fenamidone (161326-34-7) (WO 2004/058723), (3.9) fluoxastrobin (361377-29-9) (WO 2004/058723), (3.10) kresoxim-methyl (143390-89-0) (WO 2004/058723), (3.11) metominostrobin (133408-50-1) (WO 2004/058723), (3.12) orysastrobin (189892-69-1) (WO 2004/058723), (3.13) picoxystrobin (117428-22-5) (WO 2004/058723), (3.14) pyraclostrobin (175013-18-0) (WO 2004/058723), (3.15) pyrametostrobin (915410-70-7) (WO 2004/058723), (3.16) pyraoxystrobin (862588-11-2) (WO 2004/058723), (3.17) pyribencarb (799247-52-2) (WO 2004/058723), (3.18) trifloxystrobin (141517-21-7) (WO 2004/058723), (3.19) (2E)-2-(2-{[6-(3-chloro-2-methylphenoxy)-5-fluoropyrimidin-4-yl]oxy}phenyl)-2-(methoxyimino)-N-methylethanamide (WO 2004/058723), (3.20) (2E)-2-(methoxyimino)-N-methyl-2-(2-{[({(1E)-1-[3-(trifluoromethyl)phenyl]ethylidene}amino)oxy]methyl}phenyl)ethanamide (WO 2004/058723) and salts thereof., (3.21) (2E)-2-(methoxyimino)-N-methyl-2-{2-[(E)-({1-[3-(trifluoromethyl)phenyl]ethoxy}imino)methyl]phenyl}ethanamide (158169-73-4), (3.22) (2E)-2-{2-[({[(1E)-1-(3-{[(E)-1-fluoro-2-phenylethenyl]oxy}phenyl)ethylidene]amino}oxy)methyl]phenyl}-2-(methoxyimino)-N-methylethanamide (326896-28-0), (3.23) (2E)-2-{2-[({[(2E,3E)-4-(2,6-dichlorophenyl)but-3-en-2-ylidene]amino}oxy)methyl]phenyl}-2-(methoxyimino)-N-methylethanamide, (3.24) 2-chloro-N-(1,1,3-trimethyl-2,3-dihydro-1H-inden-4-yl)pyridine-3-carboxamide (119899-14-8), (3.25) 5-methoxy-2-methyl-4-(2-{[({(1E)-1-[3-(trifluoromethyl)phenyl]ethylidene}amino)oxy]methyl}phenyl)-2,4-dihydro-3H-1,2,4-triazol-3-one, (3.26) methyl (2E)-2-{2-[({cyclopropyl[(4-methoxyphenyl)imino]methyl}sulfanyl)methyl]phenyl}-3-methoxyprop-2-enoate (149601-03-6), (3.27) N-(3-ethyl-3,5,5-trimethylcyclohexyl)-3-(formylamino)-2-hydroxybenzamide (226551-21-9), (3.28) 2-{2-[(2,5-dimethylphenoxy)methyl]phenyl}-2-methoxy-N-methylacetamide (173662-97-0), (3.29) (2R)-2-{2-[(2,5-dimethylphenoxy)methyl]phenyl}-2-methoxy-N-methylacetamide (394657-24-0) and salts thereof.
(4) Inhibitors of the mitosis and cell division, for example (4.1) benomyl (17804-35-2), (4.2) carbendazim (10605-21-7), (4.3) chlorfenazole (3574-96-7), (4.4) diethofencarb (87130-20-9), (4.5) ethaboxam (162650-77-3), (4.6) fluopicolide (239110-15-7), (4.7) fuberidazole (3878-19-1), (4.8) pencycuron (66063-05-6), (4.9) thiabendazole (148-79-8), (4.10) thiophanate-methyl (23564-05-8), (4.11) thiophanate (23564-06-9), (4.12) zoxamide (156052-68-5), (4.13) 5-chloro-7-(4-methylpiperidin-1-yl)-6-(2,4,6-trifluorophenyl)[1,2,4]triazolo[1,5-a]pyrimidine (214706-53-3), (4.14) 3-chloro-5-(6-chloropyridin-3-yl)-6-methyl-4-(2,4,6-trifluorophenyl)pyridazine (1002756-87-7) and salts thereof.
(5) Compounds capable to have a multisite action, like for example (5.1) bordeaux mixture (8011-63-0), (5.2) captafol (2425-06-1), (5.3) captan (133-06-2) (WO 02/12172), (5.4) chlorothalonil (1897-45-6), (5.5) copper hydroxide (20427-59-2), (5.6) copper naphthenate (1338-02-9), (5.7) copper oxide (1317-39-1), (5.8) copper oxychloride (1332-40-7), (5.9) copper(2+) sulfate (7758-98-7), (5.10) dichlofluanid (1085-98-9), (5.11) dithianon (3347-22-6), (5.12) dodine (2439-10-3), (5.13) dodine free base, (5.14) ferbam (14484-64-1), (5.15) fluorofolpet (719-96-0), (5.16) folpet (133-07-3), (5.17) guazatine (108173-90-6), (5.18) guazatine acetate, (5.19) iminoctadine (13516-27-3), (5.20) iminoctadine albesilate (169202-06-6), (5.21) iminoctadine triacetate (57520-17-9), (5.22) mancopper (53988-93-5), (5.23) mancozeb (8018-01-7), (5.24) maneb (12427-38-2), (5.25) metiram (9006-42-2), (5.26) metiram zinc (9006-42-2), (5.27) oxine-copper (10380-28-6), (5.28) propamidine (104-32-5), (5.29) propineb (12071-83-9), (5.30) sulphur and sulphur preparations including calcium polysulphide (7704-34-9), (5.31) thiram (137-26-8), (5.32) tolylfluanid (731-27-1), (5.33) zineb (12122-67-7), (5.34) ziram (137-30-4) and salts thereof.
(6) Compounds capable to induce a host defence, like for example (6.1) acibenzolar-S-methyl (135158-54-2), (6.2) isotianil (224049-04-1), (6.3) probenazole (27605-76-1), (6.4) tiadinil (223580-51-6) and salts thereof.
(7) Inhibitors of the amino acid and/or protein biosynthesis, for example , (7.1) andoprim (23951-85-1), (7.2) blasticidin-S (2079-00-7), (7.3) cyprodinil (121552-61-2), (7.4) kasugamycin (6980-18-3), (7.5) kasugamycin hydrochloride hydrate (19408-46-9), (7.6) mepanipyrim (110235-47-7), (7.7) pyrimethanil (53112-28-0) and salts thereof.
(8) Inhibitors of the ATP production, for example (8.1) fentin acetate (900-95-8), (8.2) fentin chloride (639-58-7), (8.3) fentin hydroxide (76-87-9) and (8.4) silthiofam (175217-20-6).
(9) Inhibitors of the cell wall synthesis, for example (9.1) benthiavalicarb (177406-68-7), (9.2) dimethomorph (110488-70-5), (9.3) flumorph (211867-47-9), (9.4) iprovalicarb (140923-17-7), (9.5) mandipropamid (374726-62-2), (9.6) polyoxins (11113-80-7), (9.7) polyoxorim (22976-86-9), (9.8) validamycin A (37248-47-8), (9.9) valifenalate (283159-94-4; 283159-90-0) and (9.10) N-[2-(4-{[3-(4-chlorophenyl)prop-2-yn-1-yl]oxy}-3-methoxyphenyl)ethyl]-N2-(methylsulfonyl)valinamide (220706-93-4).
(10) Inhibitors of the lipid and membrane synthesis, for example (10.1) biphenyl (92-52-4), (10.2) chloroneb (2675-77-6), (10.3) dicloran (99-30-9), (10.4) edifenphos (17109-49-8), (10.5) etridiazole (2593-15-9), (10.6) iodocarb (55406-53-6), (10.7) iprobenfos (26087-47-8), (10.8) isoprothiolane (50512-35-1), (10.9) propamocarb (25606-41-1), (10.10) propamocarb hydrochloride (25606-41-1), (10.11) prothiocarb (19622-08-3), (10.12) pyrazophos (13457-18-6), (10.13) quintozene (82-68-8), (10.14) tecnazene (117-18-0) and (10.15) tolclofos-methyl (57018-04-9).
(11) Inhibitors of the melanine biosynthesis, for example (11.1) carpropamid (104030-54-8), (11.2) diclocymet (139920-32-4), (11.3) fenoxanil (115852-48-7), (11.4) phthalide (27355-22-2), (11.5) pyroquilon (57369-32-1) and (11.6) tricyclazole (41814-78-2).
(12) Inhibitors of the nucleic acid synthesis, for example (12.1) benalaxyl (71626-11-4), (12.2) benalaxyl-M (kiralaxyl) (98243-83-5), (12.3) bupirimate (41483-43-6), (12.4) clozylacon (67932-85-8), (12.5) dimethirimol (5221-53-4), (12.6) ethirimol (23947-60-6), (12.7) furalaxyl (57646-30-7), (12.8) hymexazol (10004-44-1), (12.9) metalaxyl (57837-19-1), (12.10) metalaxyl-M (mefenoxam) (70630-17-0), (12.11) ofurace (58810-48-3), (12.12) oxadixyl (77732-09-3) and (12.13) oxolinic acid (14698-29-4).
(13) Inhibitors of the signal transduction, for example (13.1) chlozolinate (84332-86-5), (13.2) fenpiclonil (74738-17-3), (13.3) fludioxonil (131341-86-1), (13.4) iprodione (36734-19-7), (13.5) procymidone (32809-16-8), (13.6) quinoxyfen (124495-18-7) and (13.7) vinclozolin (50471-44-8).
(14) Compounds capable to act as an uncoupler, like for example (14.1) binapacryl (485-31-4), (14.2) dinocap (131-72-6), (14.3) ferimzone (89269-64-7), (14.4) fluazinam (79622-59-6) and (14.5) meptyldinocap (131-72-6).
(15) Further compounds, like for example (15.1) benthiazole (21564-17-0), (15.2) bethoxazin (163269-30-5), (15.3) capsimycin (70694-08-5), (15.4) carvone (99-49-0), (15.5) chinomethionat (2439-01-2), (15.6) chlazafenone (688046-61-9), (15.7) cufraneb (11096-18-7), (15.8) cyflufenamid (180409-60-3), (15.9) cymoxanil (57966-95-7), (15.10) cyprosulfamide (221667-31-8), (15.11) dazomet (533-74-4), (15.12) debacarb (62732-91-6), (15.13) dichlorophen (97-23-4), (15.14) diclomezine (62865-36-5), (15.15) difenzoquat (49866-87-7), (15.16) difenzoquat methylsulphate (43222-48-6), (15.17) diphenylamine (122-39-4), (15.18) ecomate, (15.19) fenpyrazamine (473798-59-3), (15.20) flumetover (154025-04-4), (15.21) fluoroimide (41205-21-4), (15.22) flusulfamide (106917-52-6), (15.23) flutianil (304900-25-2), (15.24) fosetyl-aluminium (39148-24-8), (15.25) fosetyl-calcium, (15.26) fosetyl-sodium (39148-16-8), (15.27) hexachlorobenzene (118-74-1), (15.28) irumamycin (81604-73-1), (15.29) methasulfocarb (66952-49-6), (15.30) methyl isothiocyanate (556-61-6), (15.31) metrafenone (220899-03-6), (15.32) mildiomycin (67527-71-3), (15.33) natamycin (7681-93-8), (15.34) nickel dimethyldithiocarbamate (15521-65-0), (15.35) nitrothal-isopropyl (10552-74-6), (15.36) octhilinone (26530-20-1), (15.37) oxamocarb (917242-12-7), (15.38) oxyfenthiin (34407-87-9), (15.39) pentachlorophenol and salts (87-86-5), (15.40) phenothrin, (15.41) phosphorous acid and its salts (13598-36-2), (15.42) propamocarb-fosetylate, (15.43) propanosine-sodium (88498-02-6), (15.44) proquinazid (189278-12-4), (15.45) pyrrolnitrine (1018-71-9) (EP-A 1 559 320), (15.46) tebufloquin (376645-78-2), (15.47) tecloftalam (76280-91-6), (15.48) tolnifanide (304911-98-6), (15.49) triazoxide (72459-58-6), (15.50) trichlamide (70193-21-4), (15.51) zarilamid (84527-51-5), (15.52) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-79-6) (WO 2008013622), (15.53) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-80-9) (WO 2008013622), (15.54) 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003318-67-9) (WO 2008013622), (15.55) 1-(4-methoxyphenoxy)-3,3-dimethylbutan-2-yl 1H-imidazole-1-carboxylate (111227-17-9), (15.56) 2,3,5,6-tetrachloro-4-(methylsulfonyl)pyridine (13108-52-6), (15.57) 2,3-dibutyl-6-chlorothieno[2,3-d]pyrimidin-4(3H)-one (221451-58-7), (15.58) 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone (1003316-53-7) (WO 2008013622), (15.59) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone (1003316-54-8) (WO 2008013622), (15.60) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone (1003316-51-5) (WO 2008013622), (15.61) 2-butoxy-6-iodo-3-propyl-4H-chromen-4-one, (15.62) 2-chloro-5-[2-chloro-1-(2,6-difluoro-4-methoxyphenyl)-4-methyl-1H-imidazol-5-yl]pyridine, (15.63) 2-phenylphenol and salts (90-43-7), (15.64) 3,4,5-trichloropyridine-2,6-dicarbonitrile (17824-85-0), (15.65) 3-[5-(4-chlorophenyl)-2,3-dimethyl-1,2-oxazolidin-3-yl]pyridine, (15.66) 3-chloro-5-(4-chlorophenyl)-4-(2,6-difluorophenyl)-6-methylpyridazine, (15.67) 4-(4-chlorophenyl)-5-(2,6-difluorophenyl)-3,6-dimethylpyridazine, (15.68) 5-amino-1,3,4-thiadiazole-2-thiol, (15.69) 5-chloro-N'-phenyl-N'-(prop-2-yn-1-yl)thiophene-2-sulfonohydrazide (134-31-6), (15.70) 5-methyl-6-octyl[1,2,4]triazolo[1,5-a]pyrimidin-7-amine, (15.71) ethyl (2Z)-3-amino-2-cyano-3-phenylprop-2-enoate, (15.72) N-(4-chlorobenzyl)-3-[3-methoxy-4-(prop-2-yn-1-yloxy)phenyl]propanamide, (15.73) N-[(4-chlorophenyl)(cyano)methyl]-3-[3-methoxy-4-(prop-2-yn-1-yloxy)phenyl]propanamide, (15.74) N-[(5-bromo-3-chloropyridin-2-yl)methyl]-2,4-dichloropyridine-3-carboxamide, (15.75) N-[1-(5-bromo-3-chloropyridin-2-yl)ethyl]-2,4-dichloropyridine-3-carboxamide, (15.76) N-[1-(5-bromo-3-chloropyridin-2-yl)ethyl]-2-fluoro-4-iodopyridine-3-carboxamide, (15.77) N-{(E)-[(cyclopropylmethoxy)imino][6-(difluoromethoxy)-2,3-difluorophenyl]methyl}-2-phenylacetamide (221201-92-9), (15.78) N-{(Z)-[(cyclopropylmethoxy)imino][6-(difluoromethoxy)-2,3-difluorophenyl]methyl}-2-phenylacetamide (221201-92-9), (15.79) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide (922514-49-6) (WO 2007014290), (15.80) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide (922514-07-6) (WO 2007014290), (15.81) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide (922514-48-5) (WO 2007014290), (15.82) pentyl {6-[({[(1-methyl-1H-tetrazol-5-yl)(phenyl)methylidene]amino}oxy)methyl]pyridin-2-yl}carbamate, (15.83) phenazine-1-carboxylic acid, (15.84) quinolin-8-ol (134-31-6), (15.85) quinolin-8-ol sulfate (2:1) (134-31-6) and (15.86) (5-bromo-2-methoxy-4-methylpyridin-3-yl)(2,3,4-trimethoxy-6-methylphenyl)methanone (known from EP-A 1 559 320).

According to another object of the present invention, there is provided a method for controlling the phytopathogenic fungi of plants, crops or seeds, characterized in that an agronomically effective and substantially non-phytotoxic quantity of a pesticide composition according to the invention is applied as seed treatment, foliar application, stem application, drench or drip application (chemigation) to the seed, the plant or to the fruit of the plant or to soil or to inert substrate (e.g. inorganic substrates like sand, rockwool, glasswool; expanded minerals like perlite, vermiculite, zeolite or expanded clay), Pumice, Pyroclastic materials or stuff, synthetic organic substrates (e.g. polyurethane) organic substrates (e.g. peat, composts, tree waste products like coir, wood fibre or chips, tree bark) or to a liquid substrate (e.g. floating hydroponic systems, Nutrient Film Technique, Aeroponics) wherein the plant is growing or wherein it is desired to grow.

The expression "are applied to the plants to be treated" is understood to mean, for the purposes of the present invention, that the pesticide composition which is the subject of the invention can be applied by means of various methods of treatment such as:
- spraying onto the aerial parts of the said plants a liquid comprising one of the said compositions,
- dusting, the incorporation into the soil of granules or powders, spraying, around the said plants and in the case of trees injection or daubing,
- coating or film-coating the seeds of the said plants with the aid of a plant-protection mixture comprising one of the said compositions.

The method according to the invention can either be a curing, preventing or eradicating method.
In this method, a composition used can be prepared beforehand by mixing the two or more active compounds according to the invention.
According to an alternative of such a method, it is also possible to apply simultaneously, successively or separately compounds (A) and (B) so as to have the conjugated (A)/(B) effects, of distinct compositions each containing one of the two or three active ingredients (A) or (B).

The dose of active compound usually applied in the method of treatment according to the invention is generally and advantageously
- for foliar treatments: from 0.1 to 10,000 g/ha, preferably from 10 to 1,000 g/ha, more preferably from 50 to 300g/ha; in case of drench or drip application, the dose can even be reduced, especially while using inert substrates like rockwool or perlite;
- for seed treatment: from 2 to 200 g per 100 kilogram of seed, preferably from 3 to 150 g per 100 kilogram of seed;
- for soil treatment: from 0.1 to 10,000 g/ha, preferably from 1 to 5,000 g/ha.

The doses herein indicated are given as illustrative Examples of method according to the invention. A person skilled in the art will know how to adapt the application doses, notably according to the nature of the plant or crop to be treated.
Under specific conditions, for example according to the nature of the phytopathogenic fungus to be treated or controlled, a lower dose can offer adequate protection. Certain climatic conditions, resistance or other factors like the nature of the phytopathogenic fungi or the degree of infestation, for example, of the plants with these fungi, can require higher doses of combined active ingredients. The optimum dose usually depends on several factors, for example on the type of phytopathogenic fungus to be treated, on the type or level of development of the infested plant, on the density of vegetation or alternatively on the method of application.
Without it being limiting, the crop treated with the pesticide composition or combination according to the invention is, for example, grapevine, but this could be cereals, vegetables, lucerne, soybean, market garden crops, turf, wood, tree or horticultural plants.
The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the over-ground parts of the plant such as trunks, stems or stalks, leaves, flowers and fruit of the concerned plant.
Among the plants that can be protected by the method according to the invention, mention can be made of cotton; flax; vine; fruit or vegetable crops such as *Rosaceae sp.* (for instance pip fruit such as apples and pears, but also stone fruit such as apricots, almonds and peaches), *Ribesioidae sp., Juglandaceae sp*., *Betulaceae sp., Anacardiaceae sp., Fagaceae sp., Moraceae sp., Oleaceae sp*., *Actinidaceae sp., Lauraceae sp., Musaceae sp*. (for instance banana trees and plantins), *Rubiaceae sp*., *Theaceae sp*., *Sterculiceae sp., Rutaceae sp.* (for instance lemons oranges and grapefruit); *Solanaceae sp*. (for instance tomatoes), *Liliaceae sp., Asteraceae sp*. (for instance lettuces), *Umbelliferae sp., Cruciferae sp., Chenopodiaceae sp., Cucurbitaceae sp., Papilionaceae sp.* (for instance peas), *Rosaceae sp*. (for instance strawberries); major crops such as *Graminae sp*. (for instance maize, lawn or cereals such as wheat, rice, barley and triticale), *Asteraceae sp.* (for instance sunflower), *Cruciferae sp.* (for instance colza), *Fabacae sp*. (for instance peanuts), *Papilionaceae sp.* (for instance soybean), *Solanaceae sp*. (for instance potatoes), *Chenopodiaceae sp.* (for instance beetroots); horticultural and forest crops; as well as genetically modified homologues of these crops.

The composition according to the invention can also be used in the treatment of genetically modified organisms with the compounds according to the invention or the agrochemical compositions according to the invention. Genetically modified plants are plants into genome of which a heterologous gene encoding a protein of interest has been stably integrated. The expression "heterologous gene encoding a protein of interest" essentially means genes which give the transformed plant new agronomic properties or genes for improving the agronomic quality of the modified plant.

The composition according to the invention can also be used against fungal diseases liable to grow on or inside timber. The term "timber" means all types of species of wood and all types of working of this wood intended for construction, for example solid wood, high-density wood, laminated wood and plywood. The method for treating timber according to the invention mainly consists in contacting one or more compounds according to the invention or a composition according to the invention; this includes for example direct application, spraying, dipping, injection or any other suitable means.

Among the diseases of plants or crops that can be controlled by the method according to the invention, mention can be made of :
Powdery mildew diseases such as :
   Blumeria diseases, caused for example by *Blumeria graminis ;*
   Podosphaera diseases, caused for example by *Podosphaera leucotricha* ;
   Sphaerotheca diseases, caused for example by *Sphaerotheca fuliginea ;*
   Uncinula diseases, caused for example by *Uncinula necator;*
Rust diseases such as :
   Gymnosporangium diseases, caused for example by *Gymnosporangium sabinae ;*
   Hemileia diseases, caused for example by *Hemileia vastatrix ;*
   Phakopsora diseases, caused for example by *Phakopsora pachyrhizi* or *Phakopsora meibomiae ;*
   Puccinia diseases, caused for example by *Puccinia recondita ;*
   Uromyces diseases, caused for example by *Uromyces appendiculatus ;*
Oomycete diseases such as :
   Bremia diseases, caused for example by *Bremia lactucae ;*
   Peronospora diseases, caused for example by *Peronospora pisi* or *P. brassicae ;*
   Phytophthora diseases, caused for example by *Phytophthora infestans ;*
   Plasmopara diseases, caused for example by Plasmopara *viticola ;*
   Pseudoperonospora diseases, caused for example by *Pseudoperonospora humuli* or
*Pseudoperonospora cubensis ;*
   Pythium diseases, caused for example by *Pythium ultimum ;*
Leafspot, leaf blotch and leaf blight diseases such as :
   Alternaria diseases, caused for example by *Alternaria solani ;*
   Cercospora diseases, caused for example by *Cercospora beticola ;*
   Cladiosporum diseases, caused for example by *Cladiosporium cucumerinum ;*
   Cochliobolus diseases, caused for example by *Cochliobolus sativus ;*
   Colletotrichum diseases, caused for example by *Colletotrichum lindemuthanium ;*
   Cycloconium diseases, caused for example by *Cycloconium oleaginum ;*
   Diaporthe diseases, caused for example by *Diaporthe citri ;*
   Elsinoe diseases, caused for example by *Elsinoe fawcettii ;*
   Gloeosporium diseases, caused for example by *Gloeosporium laeticolor ;*
   Glomerella diseases, caused for example by *Glomerella cingulata ;*
   Guignardia diseases, caused for example by *Guignardia bidwelli ;*
   Leptosphaeria diseases, caused for example by *Leptosphaeria maculans ; Leptosphaeria nodorum ;*
   Magnaporthe diseases, caused for example by *Magnaporthe grisea ;*
   Mycosphaerella diseases, caused for example by *Mycosphaerella graminicola* ; Mycosphaerella arachidicola ; *Mycosphaerella fijiensis ;*
   Phaeosphaeria diseases, caused for example by *Phaeosphaeria nodorum* ;
   Pyrenophora diseases, caused for example by *Pyrenophora teres* ;
   Ramularia diseases, caused for example by *Ramularia collo-cygni* ;
   Rhynchosporium diseases, caused for example by *Rhynchosporium secalis* ;
   Septoria diseases, caused for example by *Septoria apii* or *Septoria lycopercisi* ;
   Typhula diseases, caused for example by *Typhula incarnata* ;
   Venturia diseases, caused for example by *Venturia inaequalis* ;
Root and stem diseases such as :
   Corticium diseases, caused for example by *Corticium graminearum* ;
   Fusarium diseases, caused for example by *Fusarium oxysporum* ;
   Gaeumannomyces diseases, caused for example by *Gaeumannomyces graminis* ;
   Rhizoctonia diseases, caused for example by *Rhizoctonia solani* ;
   Tapesia diseases, caused for example by *Tapesia acuformis* ;
   Thielaviopsis diseases, caused for example by *Thielaviopsis basicola* ;
Ear and panicle diseases such as :
   Alternaria diseases, caused for example by *Alternaria spp.* ;
   Aspergillus diseases, caused for example by *Aspergillus flavus* ;
   Cladosporium diseases, caused for example by *Cladosporium spp.* ;
   Claviceps diseases, caused for example by *Claviceps purpurea ;*
   Fusarium diseases, caused for example by *Fusarium culmorum ;*
   Gibberella diseases, caused for example by *Gibberella zeae ;*
   Monographelia diseases, caused for example by *Monographella nivalis ;*
Smut and bunt diseases such as:
   Sphacelotheca diseases, caused for example by *Sphacelotheca reiliana* ;
   Tilletia diseases, caused for example by *Tilletia caries ;*
   Urocystis diseases, caused for example by *Urocystis occulta ;*
   Ustilago diseases, caused for example by *Ustilago nuda ;*
Fruit rot and mould diseases such as :
   Aspergillus diseases, caused for example by *Aspergillus flavus* ;
   Botrytis diseases, caused for example by *Botrytis cinerea* ;
   Penicillium diseases, caused for example by *Penicillium expansum ;*
   Sclerotinia diseases, caused for example by *Sclerotinia sclerotiorum ;*
   Verticilium diseases, caused for example by *Verticilium alboatrum ;*
Seed and soilborne decay, mould, wilt, rot and damping-off diseases :
   Alternaria diseases, caused for example by *Alternaria brassicicola*
   Aphanomyces diseases, caused for example by *Aphanomyces euteiches*
   Ascochyta diseases, caused for example by *Ascochyta lentis*
   Aspergillus diseases, caused for example by *Aspergillus flavus*
   Cladosporium diseases, caused for example by *Cladosporium herbarum*
   Cochliobolus diseases, caused for example by *Cochliobolus sativus*
   (Conidiaform: *Drechslera, Bipolaris Syn: Helminthosporium*);
   Colletotrichum diseases, caused for example by *Colletotrichum coccodes*;
   Fusarium diseases, caused for example by *Fusarium culmorum*;
   Gibberella diseases, caused for example by *Gibberella zeae;*
   Macrophomina diseases, caused for example by *Macrophomina* phaseolina
   Monographella diseases, caused for example by *Monographella nivalis;*
   Penicillium diseases, caused for example by *Penicillium expansum*
   Phoma diseases, caused for example by *Phoma lingam*
   Phomopsis diseases, caused for example by *Phomopsis sojae;*
   Phytophthora diseases, caused for example by Phytophthora cactorum;
   Pyrenophora diseases, caused for example by *Pyrenophora graminea*
   Pyricularia diseases, caused for example by *Pyricularia oryzae*;
   Pythium diseases, caused for example by *Pythium ultimum*;
   Rhizoctonia diseases, caused for example by *Rhizoctonia solani*;
   Rhizopus diseases, caused for example by *Rhizopus oryzae*
   Sclerotium diseases, caused for example by *Sclerotium rolfsii*;
   Septoria diseases, caused for example by *Septoria nodorum*;
   Typhula diseases, caused for example by *Typhula incarnata*;
   Verticillium diseases, caused for example by *Verticillium dahliae* ;
Canker, broom and dieback diseases such as :
   Nectria diseases, caused for example by *Nectria galligena* ;
Blight diseases such as :
   Monilinia diseases, caused for example by *Monilinia laxa* ;
Leaf blister or leaf curl diseases such as :
   Taphrina diseases, caused for example by *Taphrina deformans* ;
Decline diseases of wooden plants such as :
   Esca diseases, caused for example by *Phaemoniella clamydospora* ;
   Eutypa dyeback, caused for example by *Eutypa lata* ;
   Dutch elm disease, caused for example by *Ceratocystsc ulmi* ;
Diseases of flowers and Seeds such as :
   Botrytis diseases, caused for example by *Botrytis cinerea* ;
Diseases of tubers such as :
   Rhizoctonia diseases, caused for example by *Rhizoctonia solani*
   Helminthosporium diseases, caused for example by *Helminthosporium solani.*

The compounds according to the invention can also be used for the preparation of composition useful to curatively or preventively treat human or animal fungal diseases such as, for example, mycoses, dermatoses, trichophyton diseases and candidiases or diseases caused by *Aspergillus spp.,* for example *Aspergillus fumigatus.*

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.
Among the plants that can be protected by the method according to the invention, mention may be made of major field crops like corn, soybean, cotton, *Brassica* oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., O*/*eaceae sp., Actinidaceae sp., Lauraceae sp., Musaceae sp*. (for instance banana trees and plantings), *Rubiaceae sp.* (for instance coffee), *Theaceae sp., Sterculiceae sp., Rutaceae sp.* (for instance lemons, oranges and grapefruit) ; *Solanaceae sp.* (for instance tomatoes, potatoes, peppers, eggplant), *Liliaceae sp.,* Compositiae *sp*. (for instance lettuce, artichoke and chicory - including root chicory, endive or common chicory), *Umbelliferae sp.* (for instance carrot, parsley, celery and celeriac), *Cucurbitaceae sp.* (for instance cucumber- including pickling cucumber, squash, watermelon, gourds and melons), *Alliaceae* sp. (for instance onions and leek), Cruciferae *sp*. (for instance white cabbage, red cabbage, broccoli, cauliflower, brussel sprouts, pak choi, kohlrabi, radish, horseradish, cress, Chinese cabbage), Leguminosae *sp*. (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology or RNA interference - RNAi - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted microorganisms. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted microorganisms, the treated plants display a substantial degree of resistance to these microorganisms. In the present case, unwanted microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).
Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Examples of nematode resistant plants are described in e.g. US Patent Application Nos 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 or 12/497,221.
Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.
Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.
Examples of plants with the above-mentioned traits are non-exhaustively listed in Table A.

Plants that may be treated according to the invention are hybrid plants that already express the characteristic of heterosis or hybrid vigor which results in generally higher yield, vigor, health and resistance towards biotic and abiotic stresses). Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (WO 92/05251, WO 95/09910, WO 98/27806, WO 05/002324, WO 06/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 89/10396 in which, for example, a ribonuclease such as barnase is selectively expressed in the tapetum cells in the stamens. Fertility can then be restored by expression in the tapetum cells of a ribonuclease inhibitor such as barstar (e.g. WO 91/02069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.
Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium Salmonella *typhimurium* (Comai et al., 1983, Science 221, 370-371), the CP4 gene of the bacterium *Agrobacterium sp.* (Barry et al., 1992, Curr. Topics Plant Physiol. 7, 139-145), the genes encoding a Petunia EPSPS (Shah et al., 1986, Science 233, 478-481), a Tomato EPSPS (Gasser et al., 1988, J. Biol. Chem. 263, 4280-4289), or an Eleusine EPSPS (WO 01/66704). It can also be a mutated EPSPS as described in for example EP 0837944, WO 00/66746, WO 00/66747 or WO02/26995. Glyphosate-tolerant plants can also be obtained by expressing a gene that encodes a glyphosate oxido-reductase enzyme as described in U.S. Patent Nos. 5,776,760 and 5,463,175. Glyphosate-tolerant plants can also be obtained by expressing a gene that encodes a glyphosate acetyl transferase enzyme as described in for example WO 02/36782, WO 03/092360, WO 05/012515 and WO 07/024782. Glyphosate-tolerant plants can also be obtained by selecting plants containing naturally-occurring mutations of the above-mentioned genes, as described in for example WO 01/024615 or WO 03/013226. Plants expressing EPSPS genes that confer glyphosate tolerance are described in e.g. US Patent Application Nos 11/517,991, 10/739,610, 12/139,408, 12/352,532, 11/312,866, 11/315,678, 12/421,292, 11/400,598, 11/651,752, 11/681,285, 11/605,824, 12/468,205, 11/760,570, 11/762,526, 11/769,327, 11/769,255, 11/943801 or 12/362,774. Plants comprising other genes that confer glyphosate tolerance, such as decarboxylase genes, are described in e.g. US patent applications 11/588,811, 11/185,342, 12/364,724, 11/185,560 or 12/423,926.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition, e.g. described in US Patent Application No 11/760,602. One such efficient detoxifying enzyme is an enzyme encoding a phosphinothricin acetyltransferase (such as the bar or pat protein from Streptomyces species). Plants expressing an exogenous phosphinothricin acetyltransferase are for example described in U.S. Patent Nos. 5,561,236; 5,648,477; 5,646,024; 5,273,894; 5,637,489; 5,276,268; 5,739,082; 5,908,810 and 7,112,665.
Further herbicide-tolerant plants are also plants that are made tolerant to the herbicides inhibiting the enzyme hydroxyphenylpyruvatedioxygenase (HPPD). Hydroxyphenylpyruvatedioxygenases are enzymes that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated or chimeric HPPD enzyme as described in WO 96/38567, WO 99/24585, WO 99/24586, WO 2009/144079, WO 2002/046387, or US 6,768,044.. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme having prephenate deshydrogenase (PDH) activity in addition to a gene encoding an HPPD-tolerant enzyme, as described in WO 2004/024928. Further, plants can be made more tolerant to HPPD-inhibitor herbicides by adding into their genome a gene encoding an enzyme capable of metabolizing or degrading HPPD inhibitors, such as the CYP450 enzymes shown in WO 2007/103567 and WO 2008/150473.
Still further herbicide resistant plants are plants that are made tolerant to acetolactate synthase (ALS) inhibitors. Known ALS-inhibitors include, for example, sulfonylurea, imidazolinone, triazolopyrimidines, pryimidinyoxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright (2002, Weed Science 50:700-712), but also, in U.S. Patent No. 5,605,011, 5,378,824, 5,141,870, and 5,013,659. The production of sulfonylurea-tolerant plants and imidazolinone-tolerant plants is described in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 07/024782 and US Patent Application No 61/288958.

Other plants tolerant to imidazolinone and/or sulfonylurea can be obtained by induced mutagenesis, selection in cell cultures in the presence of the herbicide or mutation breeding as described for example for soybeans in U.S. Patent 5,084,082, for rice in WO 97/41218, for sugar beet in U.S. Patent 5,773,702 and WO 99/057965, for lettuce in U.S. Patent 5,198,599, or for sunflower in WO 01/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.
An "insect-resistant transgenic plant", as used herein, includes any plant containing at least one transgene comprising a coding sequence encoding:
1) an insecticidal crystal protein from *Bacillus thuringiensis* or an insecticidal portion thereof, such as the insecticidal crystal proteins listed by Crickmore et al. (1998, Microbiology and Molecular Biology Reviews, 62: 807-813), updated by Crickmore et al. (2005) at the *Bacillus thuringiensis* toxin nomenclature, online at:
   http://www.lifesci.sussex.ac.uk/Home/Neil_Crickmore/Bt/), or insecticidal portions thereof, e.g., proteins of the Cry protein classes Cry1Ab, Cry1Ac, Cry1B, Cry1C, Cry1D, Cry1F, Cry2Ab, Cry3Aa, or Cry3Bb or insecticidal portions thereof (e.g. EP 1999141 and WO 2007/107302), or such proteins encoded by synthetic genes as e.g. described in and US Patent Application No 12/249,016 ; or
2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins (Moellenbeck et al. 2001, Nat. Biotechnol. 19: 668-72; Schnepf et al. 2006, Applied Environm. Microbiol. 71, 1765-1774) or the binary toxin made up of the Cry1A or Cry1 F proteins and the Cry2Aa or Cry2Ab or Cry2Ae proteins (US Patent Appl. No. 12/214,022 and EP 08010791.5); or
3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A.105 protein produced by corn event MON89034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or
5) an insecticidal secreted protein from *Bacillus thuringiensis* or *Bacillus cereus,* or an insecticidal portion thereof, such as the vegetative insecticidal (VIP) proteins listed at:
   http://www.lifesci.sussex.ac.uk/home/Neil_Crickmore/Bt/vip.html, e.g., proteins from the VIP3Aa protein class; or
6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins (WO 94/21795); or
7) a hybrid insecticidal protein comprising parts from different secreted proteins from *Bacillus thuringiensis* or *Bacillus cereus,* such as a hybrid of the proteins in 1) above or a hybrid of the proteins in 2) above; or
8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102; or
9) a secreted protein from *Bacillus thuringiensis* or *Bacillus cereus* which is insecticidal in the presence of a crystal protein from *Bacillus thuringiensis,* such as the binary toxin made up of VIP3 and Cry1A or Cry1 F (US Patent Appl. No. 61/126083 and 61/195019), or the binary toxin made up of the VIP3 protein and the Cry2Aa or Cry2Ab or Cry2Ae proteins (US Patent Appl. No. 12/214,022 and EP 08010791.5).
10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein)

Of course, an insect-resistant transgenic plant, as used herein, also includes any plant comprising a combination of genes encoding the proteins of any one of the above classes 1 to 10. In one embodiment, an insect-resistant plant contains more than one transgene encoding a protein of any one of the above classes 1 to 10, to expand the range of target insect species affected when using different proteins directed at different target insect species, or to delay insect resistance development to the plants by using different proteins insecticidal to the same target insect species but having a different mode of action, such as binding to different receptor binding sites in the insect.

An "insect-resistant transgenic plant", as used herein, further includes any plant containing at least one transgene comprising a sequence producing upon expression a double-stranded RNA which upon ingestion by a plant insect pest inhibits the growth of this insect pest, as described e.g. in WO 2007/080126, WO 2006/129204, WO 2007/074405, WO 2007/080127 and WO 2007/035650.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
1) plants which contain a transgene capable of reducing the expression and/or the activity of poly(ADP-ribose) polymerase (PARP) gene in the plant cells or plants as described in WO 00/04173, WO/2006/045633, EP 04077984.5, or EP 06009836.5.
2) plants which contain a stress tolerance enhancing transgene capable of reducing the expression and/or the activity of the PARG encoding genes of the plants or plants cells, as described e.g. in WO 2004/090140.
3) plants which contain a stress tolerance enhancing transgene coding for a plant-functional enzyme of the nicotineamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP 04077624.7, WO 2006/133827, PCT/EP07/002433, EP 1999263, or WO 2007/107326.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP 0571427, WO 95/04826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, EP 06090134.5, EP 06090228.5, EP 06090227.7, EP 07090007.1, EP 07090009.7, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026, WO 97/20936
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP 0663956, WO 96/01904, WO 96/21023, WO 98/39460, and WO 99/24593, plants producing alpha-1,4-glucans as disclosed in WO 95/31553, US 2002031826, US 6,284,479, US 5,712,107, WO 97/47806, WO 97/47807, WO 97/47808 and WO 00/14249, plants producing alpha-1,6 branched alpha-1,4-glucans, as disclosed in WO 00/73422, plants producing alternan, as disclosed in e.g. WO 00/47727, WO 00/73422, EP 06077301.7, US 5,908,975 and EP 0728213,
3) transgenic plants which produce hyaluronan, as for example disclosed in WO 2006/032538, WO 2007/039314, WO 2007/039315, WO 2007/039316, JP 2006304779, and WO 2005/012529.
4) transgenic plants or hybrid plants, such as onions with characteristics such as 'high soluble solids content', 'low pungency' (LP) and/or `long storage' (LS), as described in US Patent Appl. No. 12/020,360 and 61/054,026.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes as described in WO 98/00549
b) Plants, such as cotton plants, containing an altered form of rsw2 or rsw3 homologous nucleic acids as described in WO 2004/053219
c) Plants, such as cotton plants, with increased expression of sucrose phosphate synthase as described in WO 01/17333
d) Plants, such as cotton plants, with increased expression of sucrose synthase as described in WO 02/45485
e) Plants, such as cotton plants, wherein the timing of the plasmodesmatal gating at the basis of the fiber cell is altered, e.g. through downregulation of fiber-selective β-1,3-glucanase as described in WO 2005/017157, or as described in EP 08075514.3 or US Patent Appl. No. 61/128,938
f) Plants, such as cotton plants, having fibers with altered reactivity, e.g. through the expression of N-acetylglucosaminetransferase gene including nodC and chitin synthase genes as described in WO 2006/136351

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics and include:
a) Plants, such as oilseed rape plants, producing oil having a high oleic acid content as described e.g. in US 5,969,169, US 5,840,946 or US 6,323,392 or US 6,063,947
b) Plants such as oilseed rape plants, producing oil having a low linolenic acid content as described in US 6,270,828, US 6,169,190, or US 5,965,755
c) Plant such as oilseed rape plants, producing oil having a low level of saturated fatty acids as described e.g. in US Patent No. 5,434,283 or US Patent Application No 12/668303

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering as described in US Patent Appl. No. 61/135,230 WO09/068313 and WO10/006732.
Particularly useful transgenic plants which may be treated according to the invention are plants containing transformation events, or combination of transformation events, that are the subject of petitions for non-regulated status, in the United States of America, to the Animal and Plant Health Inspection Service (APHIS) of the United States Department of Agriculture (USDA) whether such petitions are granted or are still pending. At any time this information is readily available from APHIS (4700 River Road Riverdale, MD 20737, USA), for instance on its internet site (URL http://www.aphis.usda.gov/brs/not_reg.html). On the filing date of this application the petitions for nonregulated status that were pending with APHIS or granted by APHIS were those listed in table B which contains the following information:
- Petition : the identification number of the petition. Technical descriptions of the transformation events can be found in the individual petition documents which are obtainable from APHIS, for example on the APHIS website, by reference to this petition number. These descriptions are herein incorporated by reference.
- Extension of Petition : reference to a previous petition for which an extension is requested.
- Institution : the name of the entity submitting the petition.
- Regulated article : the plant species concerned.
- Transgenic phenotype : the trait conferred to the plants by the transformation event.
- Transformation event or line : the name of the event or events (sometimes also designated as lines or lines) for which nonregulated status is requested.
- APHIS documents : various documents published by APHIS in relation to the Petition and which can be requested with APHIS.

Additional particularly useful plants containing single transformation events or combinations of transformation events are listed for example in the databases from various national or regional regulatory agencies (see for example http://gmoinfo.jrc.it/gmp browse.aspx and http://www.agbios.com/dbase.php).

Further particularly transgenic plants include plants containing a transgene in an agronomically neutral or beneficial position as described in any of the patent publications listed in Table C.

**Table A**

| Trait | Reference | |
|---|---|---|
| **Water use efficiency** | WO2000/073475 | |
| **Nitrogen use efficiency** | WO1995/009911 | WO2007/076115 |
| | WO1997/030163 | WO 2005/103270 |
| | WO2007/092704 | WO2002/002776 |
| **Improved photosynthesis** | WO2008/056915 | WO2004/101751 |
| **Nematode resistance** | WO 1995/020669 | WO2003/033651 |
| | WO2001/051627 | WO1999/060141 |
| | WO2008/139334 | WO1998/012335 |
| | WO2008/095972 | WO1996/030517 |
| | WO2006/085966 | WO1993/018170 |
| **Reduced pod dehiscence** | WO2006/009649 | WO1997/013865 |
| | WO2004/113542 | WO 1996/030529 |
| | WO1999/015680 | WO1994/023043 |
| | WO1999/000502 | |
| **Aphid resistance** | WO2006/125065 | WO2008/067043 |
| | WO1997/046080 | WO2004/072109 |
| **Sclerotinia resistance** | WO2006/135717 | WO2005/000007 |
| | WO2006/055851 | WO2002/099385 |
| | WO2005/090578 | WO2002/061043 |
| **Botrytis resistance** | WO2006/046861 | WO2002/085105 |
| **Bremia resistance** | US 20070022496 | WO2004/049786 |
| | WO2000/063432 | |
| **Erwinia resistance** | WO2004/049786 | |
| **Closterovirus resistance** | WO2007/073167 | WO2002/022836 |
| | WO2007/053015 | |
| **Stress tolerance (including** | WO2010/019838 | WO2008/00248 |
| **drought tolerance)** | WO2009/049110 | WO2005/033318 |
| **Tobamovirus resistance** | WO2006/038794 | |

**Table B**

| | | | | | |
|---|---|---|---|---|---|
| **Petitions of Nonregulated Status Granted or Pending by APHIS as of March 31, 2010** | | | | | |
| NOTE: To obtain the most up-to-date list of Crops No Longer Regulated, please look at the Current Status of Petitions. This list is automatically updated and reflects all petitions received to date by APHIS, including petitions pending, withdrawn, or approved. | | | | | |
| **Abbreviations:** CMV-cucumber mosaic virus; CPB-colorado potato beetle; PLRV- potato leafroll virus; PRSV-papaya ringspot virus; PVY-potato virus Y; WMV2- watermelon mosaic virus 2 ZYMV-zucchini yellow mosaic virus | | | | | |
| | | | | | |

| **Petitions for Nonregulated Status Pending** | | | | | |
|---|---|---|---|---|---|
| **Applicant Documents** | | | | | |
| **Petition** | **Extension of Petition Number ***** | **Institution** | **Regulated Article** | **Transgenic Phenotype** | **Transformation Event or Line** |
| 10-070-01p | | Virginia Tech | Peanut | Sclerotinia blight resistant | N70, P39, and W171 |
| 09-349-01p | | Dow AgroSciences | Soybean | Herbicide Tolerant | DAS-68416-4 |
| 09-328-01p | | Bayer Crop Science | Soybean | Herbicide Tolerant | FG72 |
| 09-233-01p | | Dow | Corn | Herbicide Tolerant | DAS-40278-9 |
| 09-201-01p | | Monsanto | Soybean | | MON-87705-6 |
| 09-183-01p | | Monsanto | Soybean | | MON-87769 |
| 09-082-01p | | Monsanto | Soybean | Lepidopteran resistant | MON 87701 |
| 09-063-01p | | Stine Seed | Corn | Glyphosate tolerant | HCEM485 |
| 09-055-01p | | Monsanto | Corn | Drought Tolerant | MON 87460 |
| 09-015-01p | | BASF Plant Science, LLC | Soybean | Herbicide Tolerant | BPS-CV127-9 Soybean |
| 08-366-01p | | ArborGen | Eucalyptu s | Freeze Tolerant, Fertility Altered | ARB-FTE1-08 |
| 08-340-01p | | Bayer | Cotton | Glufosinate Tolerant, Insect Resistant | T304-40XGHB119 |
| 08-338-01p | | Pioneer | Corn | Male Sterile, Fertility Restored, Visual Marker | DP-32138-1 |
| 08-315-01p | | Florigene | Rose | Altered Flower Color | IFD-52401-4 and IFD-529Ø1-9 |
| 07-253-01p | | Syngenta | Corn | Lepidopteran resistant | MIR-162 Maize |
| 07-108-01p | | Syngenta | Cotton | Lepidopteran Resistant | COT67B |
| 06-354-01p | | Pioneer | Soybean | High Oleic Acid | DP-3Ø5423-1 |
| 05-280-01p | | Syngenta | Corn | Thermostable alpha-amylase | 3272 |
| 04-110-01p | | Monsanto & Forage Genetics | Alfalfa | Glyphosate Tolerant | J101, J163 |
| 03-104-01p | | Monsanto & Scotts | Creeping bentgrass | Glyphosate Tolerant | ASR368 |
| | | | | | |

| **Petitions for Nonregulated Status Granted** | | | | | |
|---|---|---|---|---|---|
| **Applicant Documents** | | | | | |
| **Petition** | **Extension of Petition Number ***** | **Institution** | **Regulated Article** | **Transgenic Phenotype** | **Transformation Event or Line** |
| 07-152-01p | | Pioneer | Corn | glyphosate & Imidazolinone tolerant | DP-098140-6 |
| 04-337-01p | | University of Florida | Papaya | Papaya Ringspot Virus Resistant | X17-2 |
| 06-332-01p | | Bayer CropScience | Cotton | Glyphosate tolerant | GHB614 |
| 06-298-01p | | Monsanto | Corn | European Corn Borer resistant | MON 89034 |
| 06-271-01p | | Pioneer | Soybean | Glyphosate & acetolactate synthase tolerant | 356043 (DP-356043-5) |
| 06-234-01p | 98-329-01p | Bayer CropScience | Rice | Phosphinothricin tolerant | LLRICE601 |
| 06-178-01p | | Monsanto | Soybean | Glyphosate tolerant | MON 89788 |
| 04-362-01p | | Syngenta | Corn | Corn Rootworm Protected | MIR604 |
| 04-264-01p | | ARS | Plum | Plum Pox Virus Resistant | C5 |
| 04-229-01p | | Monsanto | Corn | High Lysine | LY038 |
| 04-125-01p | | Monsanto | Corn | Corn Rootworm Resistant | 88017 |
| 04-086-01p | | Monsanto | Cotton | Glyphosate Tolerant | MON 88913 |
| 03-353-01p | | Dow | Corn | Corn Rootworm Resistant | 59122 |
| 03-323-01p | | Monsanto | Sugar Beet | Glyphosate Tolerant | H7-1 |
| 03-181-01p | 00-136-01 p | Dow | Corn | Lepidopteran Resistant & Phosphinothricin tolerant | TC-6275 |
| 03-155-01p | | Syngenta | Cotton | Lepidopteran Resistant | COT 102 |
| 03-036-01p | | Mycogen/Dow | Cotton | Lepidopteran Resistant | 281-24-236 |
| 03-036-02p | | Mycogen/Dow | Cotton | Lepidopteran Resistant | 3006-210-23 |
| 02-042-01p | | Aventis | Cotton | Phosphinothericin tolerant | LLCotton25 |
| 01-324-01p | 98-216-01 p | Monsanto | Rapesee d | Glyphosate tolerant | RT200 |
| 01-206-01p | 98-278-01p | Aventis | Rapesee d | Phosphinothricin tolerant & pollination control | MS1 & RF1/RF2 |
| 01-206-02p | 97-205-01p | Aventis | Rapesee d | Phosphinothricin tolerant | Topas 19/2 |
| 01-137-01p | | Monsanto | Corn | Corn Rootworm Resistant | MON 863 |
| 01-121-01p | | Vector | Tobacco | Reduced nicotine | Vector 21-41 |
| 00-342-01p | | Monsanto | Cotton | Lepidopteran resistant | Cotton Event 15985 |
| 00-136-01p | | Mycogen c/o Dow & Pioneer | Corn | Lepidopteran resistant phosphinothricin tolerant | Line 1507 |
| 00-011-01p | 97-099-01p | Monsanto | Corn | Glyphosate tolerant | N K603 |
| 99-173-01p | 97-204-01p | Monsanto | Potato | PLRV & CPB resistant | RBMT22-82 |
| 98-349-01p | 95-228-01p | AgrEvo | Corn | Phosphinothricin tolerant and Male sterile | MS6 |
| 98-335-01p | | U. of Saskatchewan | Flax | Tolerant to soil residues of sulfonyl urea herbicide | CDC Triffid |
| 98-329-01p | | AgrEvo | Rice | Phosphinothricin tolerant | LLRICE06, LLRICE62 |
| 98-278-01p | | AgrEvo | Rapesee d | Phosphinothricin tolerant & Pollination control | MS8 & RF3 |
| 98-238-01p | | AgrEvo | Soybean | Phosphinothricin tolerant | GU262 |
| 98-216-01p | | Monsanto | Rapesee d | Glyphosate tolerant | RT73 |
| 98-173-01p | | Novartis Seeds & Monsanto | Beet | Glyphosate tolerant | GTSB77 |
| 98-014-01p | 96-068-01p | AgrEvo | Soybean | Phosphinothricin tolerant | A5547-127 |
| 97-342-01p | | Pioneer | Corn | Male sterile & Phosphinothricin tolerant | 676, 678, 680 |
| 97-339-01p | | Monsanto | Potato | CPB & PVY resistant | RBMT15-101, SEMT15-02, SEMT15-15 |
| 97-336-01p | | AgrEvo | Beet | Phosphinothricin tolerant | T-120-7 |
| 97-287-01p | | Monsanto | Tomato | Lepidopteran resistant | 5345 |
| 97-265-01p | | AgrEvo | Corn | Phosphinothricin tolerant & Lep. resistant | CBH-351 |
| 97-205-01p | | AgrEvo | Rapesee d | Phosphinothricin tolerant | T45 |
| 97-204-01p | | Monsanto | Potato | CPB & PLRV resistant | RBMT21-129 & RBMT21-350 |
| 97-148-01p | | Bejo | Cichoriu m intybus | Male sterile | RM3-3, RM3-4, RM3-6 |
| 97-099-01p | | Monsanto | Corn | Glyphosate tolerant | GA21 |
| 97-013-01p | | Calgene | Cotton | Bromoxynil tolerant & Lepidopteran resistant | Events 31807 & 31808 |
| 97-008-01p | | Du Pont | Soybean | Oil profile altered | G94-1, G94-19, G-168 |
| 96-317-01p | | Monsanto | Corn | Glyphosate tolerant & ECB resistant | MON802 |
| 96-291-01p | | DeKalb | Corn | European Corn Borer resistant | DBT418 |
| 96-248-01p | 92-196-01 p | Calgene | Tomato | Fruit ripening altered | 1 additional FLAVRSAVR line |
| 96-068-01p | | AgrEvo | Soybean | Phosphinothricin tolerant | W62, W98, A2704-12, A2704-21, A5547-35 |
| 96-051-01p | | Cornell U | Papaya | PRSV resistant | 55-1, 63-1 |
| 96-017-01p | 95-093-01p | Monsanto | Corn | European Corn Borer resistant | MON809 & MON810 |
| 95-352-01p | | Asgrow | Squash | CMV, ZYMV, WMV2 resistant | CZW-3 |
| 95-338-01p | | Monsanto | Potato | CPB resistant | SBT02-5 & -7, ATBT04-6 &-27, -30, -31, -36 |
| 95-324-01p | | Agritope | Tomato | Fruit ripening altered | 35 1 N |
| 95-256-01p | | Du Pont | Cotton | Sulfonylurea tolerant | 19-51 a |
| 95-228-01p | | Plant Genetic Systems | Corn | Male sterile | MS3 |
| 95-195-01p | | Northrup King | Corn | European Corn Borer resistant | Bt11 |
| 95-179-01p | 92-196-01 p | Calgene | Tomato | Fruit ripening altered | 2 additional FLAVRSAVR lines |
| 95-145-01p | | DeKalb | Corn | Phosphinothricin tolerant | B16 |
| 95-093-01p | | Monsanto | Corn | Lepidopteran resistant | MON 80100 |
| 95-053-01p | | Monsanto | Tomato | Fruit ripening altered | 8338 |
| 95-045-01p | | Monsanto | Cotton | Glyphosate tolerant | 1445, 1698 |
| 95-030-01p | 92-196-01p | Calgene | Tomato | Fruit ripening altered | 20 additional FLAVRSAVR lines |
| 94-357-01p | | AgrEvo | Corn | Phosphinothricin tolerant | T14, T25 |
| 94-319-01p | | Ciba Seeds | Corn | Lepidopteran resistant | Event 176 |
| 94-308-01p | | Monsanto | Cotton | Lepidopteran resistant | 531, 757, 1076 |
| 94-290-01p | | Zeneca & Petoseed | Tomato | Fruit polygalacturonase level decreased | B, Da, F |
| 94-257-01p | | Monsanto | Potato | Coleopteran resistant | BT6, BT10, BT12, BT16, BT17, BT18, BT23 |
| 94-230-01p | 92-196-01p | Calgene | Tomato | Fruit ripening altered | 9 additional FLAVRSAVR lines |
| 94-228-01p | | DNA Plant Tech | Tomato | Fruit ripening altered | 1345-4 |
| 94-227-01p | 92-196-01p | Calgene | Tomato | Fruit ripening altered | Line N73 1436-111 |
| 94-090-01p | | Calgene | Rapesee d | Oil profile altered | pCGN3828-212/86- 18 & 23 |
| 93-258-01p | | Monsanto | Soybean | Glyphosate tolerant | 40-3-2 |
| 93-196-01p | | Calgene | Cotton | Bromoxynil tolerant | BXN |
| 92-204-01p | | Upjohn | Squash | WMV2 & ZYMV resistant | ZW-20 |
| 92-196-01p | | Calgene | Tomato | Fruit ripening altered | FLAVR SAVR |
| ***** Extension of Petition Number: Under 7CFR 340.6(e) a person may request that APHIS extend a determination of non-regulated status to other organisms based on their similarity of the previously deregulated article. This column lists the previously granted petition of that degregulated article.** | | | | | |
| ****** Preliminary EA: The Environmental Assessment initially available for Public comment prior to finalization.** | | | | | |

**Table C**

| Plant species | Event | Trait | Patent reference |
|---|---|---|---|
| **Corn** | **PV-ZMGT32 (NK603)** | **Glyphosate tolerance** | US 2007-056056 |
| **Corn** | **MIR604** | **Insect resistance (Cry3a055)** | EP 1 737 290 |
| **Corn** | **LY038** | **High lysine content** | US 7,157,281 |
| **Corn** | **3272** | **Self processing corn (alpha-amylase)** | US 2006-230473 |
| **Corn** | **TC1507** | **Insect resistance (Cry1F)** | US 7,435,807 |
| **Corn** | **MON810** | **Insect resistance (Cry1Ab)** | US2004-180373 |
| **Corn** | **VIP1034** | **Insect resistance** | WO 03/052073 |
| **Corn** | **B16** | **Glufosinate resistance** | US 2003-126634 |
| **Corn** | **GA21** | **Glyphosate resistance** | US 6,040,497 |
| **Corn** | **GG25** | **Glyphosate resistance** | US 6,040,497 |
| **Corn** | **GJ11** | **Glyphosate resistance** | US 6,040,497 |
| **Corn** | **FI117** | **Glyphosate resistance** | US 6,040,497 |
| **Corn** | **GAT-ZM1** | **Glufosinate tolerance** | WO 01/51654 |
| **Corn** | **MON87460** | **Drought tolerance** | WO 2009/111263 |
| **Corn** | **DP-098140-6** | **Glyphosate tolerance / ALS inhibitor tolerance** | WO 2008/112019 |
| **Wheat** | **Event 1** | **Fusarium resistance (trichothecene 3-O-acetyltransferase)** | CA 2561992 |
| **Sugar beet** | **T227-1** | **Glyphosate tolerance** | US 2004-117870 |
| **Sugar beet** | **H7-1** | **Glyphosate tolerance** | WO 2004-074492 |
| **Soybean** | **MON89788** | **Glyphosate tolerance** | US 2006-282915 |
| **Soybean** | **A2704-12** | **Glufosinate tolerance** | WO 2006/108674 |
| **Soybean** | **A5547-35** | **Glufosinate tolerance** | WO 2006/108675 |
| **Soybean** | **DP-305423-1** | **High oleic acid / ALS inhibitor tolerance** | WO 2008/054747 |
| **Rice** | **GAT-OS2** | **Glufosinate tolerance** | WO 01/83818 |
| **Rice** | **GAT-OS3** | **Glufosinate tolerance** | US 2008-289060 |
| **Rice** | **PE-7** | **Insect resistance (Cry1Ac)** | WO 2008/114282 |
| **Oilseed rape** | **MS-B2** | **Male sterility** | WO 01/31042 |
| **Oilseed rape** | **MS-BN1/RF-BN1** | **Male sterility/restoration** | WO 01/41558 |
| **Oilseed rape** | **RT73** | **Glyphosate resistance** | WO 02/36831 |
| **Cotton** | **CE43-67B** | **Insect resistance (Cry1Ab)** | WO 2006/128573 |
| **Cotton** | **CE46-02A** | **Insect resistance (Cry1Ab)** | WO 2006/128572 |
| **Cotton** | **CE44-69D** | **Insect resistance (Cry1Ab)** | WO 2006/128571 |
| **Cotton** | **1143-14A** | **Insect resistance (Cry1Ab)** | WO2006/128569 |
| **Cotton** | **1143-51B** | **Insect resistance (Cry1Ab)** | WO2006/128570 |
| **Cotton** | **T342-142** | **Insect resistance (Cry1Ab)** | WO2006/128568 |
| **Cotton** | **event3006-210-23** | **Insect resistance (Cry1Ac)** | WO2005/103266 |
| **Cotton** | **PV-GHGT07 (1445)** | **Glyphosate tolerance** | US 2004-148666 |
| **Cotton** | **MON88913** | **Glyphosate tolerance** | WO2004/072235 |
| **Cotton** | **EE-GH3** | **Glyphosate tolerance** | WO2007/017186 |
| **Cotton** | **T304-40** | **Insect-resistance (Cry1Ab)** | WO2008/122406 |
| **Cotton** | **Cot202** | **Insect resistance (VIP3)** | US 2007-067868 |
| **Cotton** | **LLcotton25** | **Glufosinate resistance** | WO2007/017186 |
| **Cotton** | **EE-GH5** | **Insect resistance (Cry1Ab)** | WO2008/122406 |
| **Cotton** | **event 281-24-236** | **Insect resistance (Cry1F)** | WO2005/103266 |
| **Cotton** | **Cot102** | **Insect resistance (Vip3A)** | US 2006-130175 |
| **Cotton** | **MON 15985** | **Insect resistance (Cry1A/Cry2Ab)** | US2004-250317 |
| **Bent Grass** | **Asr-368** | **Glyphosate tolerance** | US 2006-162007 |
| **Brinjal** | **EE-1** | **Insect resistance (Cry1Ac)** | WO 2007/091277 |

The following table 1 illustrates in a non limiting manner examples of compounds according to the invention.

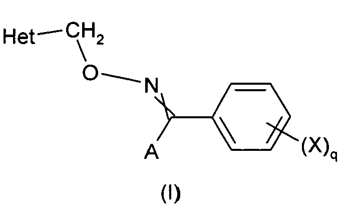

In table 1, we use the following abbreviations for specified claimed elements "A" and "Het" of the generic structure (I) of the invention:

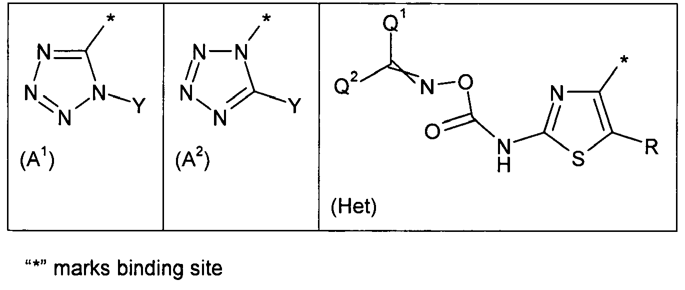

**Table 1**

| **Example** | **A** | **Het** | **Q¹** | **Q²** | **R** | **Phenyl-(X)_{q}** | **Y** | **log P** | **MW measured** |
|---|---|---|---|---|---|---|---|---|---|
| 1 | (A¹) | (Het) | H | phenyl | H | phenyl | CH₃ | 3.13 | 342 |

The following examples illustrate in a non-limiting manner the preparation and efficacy of the compounds of formula (I) according to the invention.

### Preparation example 1: ({4-[({[(Z)-(1-methyl-1H-tetrazol-5-yl)(phenyl)methylene]amino}oxy)methyl]-1,3-thiazol-2-yl}amino)({[(E)-phenylmethylene]amino}oxy)methanone (compound 1) according to process P1

### Step 1:

To a stirred solution of (4-[({[(Z)-(1-methyl-1H-tetrazol-5-yl)(phenyl)methylene]amino}oxy)methyl]-1,3-thiazol-2-amine (600 mg, 1.90 mmol) in dichloromethane (5 mL), was added at 0°C triethylamine (1.60 mL, 11.41 mmol). A solution of Bis(trichloromethyl) carbonate in dichloromethane (5 mL) was added dropwise to the reaction mixture at 0°C. After 30 minutes of stirring at 0°C, the reaction mixture was allowed to room temperature for two hours and was then warmed to reflux for 4 hours and left overnight at room temperature. The reaction mixture was washed successively with water and brine. After separation, the organic phase was dried over magnesium sulfate, filtered and evaporated *in vacuo* to yield (Z)-N-[(2-isocyanato-1,3-thiazol-4-yl)methoxy]-1-(1-methyl-1H-tetrazol-5-yl)-1-phenylmethanimine which will be used without further purification [437mg, yield 73% ; HPLC/MS : m/z = 342 (M+H) ; logP_{(HCOOH)} = 3.13].

### Step 2:

To a stirred solution of (E)-N-hydroxy-1-phenylmethanimine (113 mg, 0.937 mmol) in tetrahydrofuran (2 mL), was added at room temperature 1,8-Diazabicyclo(5.4.0)undec-7-ene (36 mg, 0.234 mmol). A solution of (Z)-N-[(2-isocyanato-1,3-thiazol-4-yl)methoxy]-1-(1-methyl-1H-tetrazol-5-yl)-1-phenylmethanimine (160 mg, 0.469 mmol) in tetrahydrofuran (3 mL) was added to the reaction mixture. After two hours of stirring at room temperature, the reaction mixture was evaporated *in vacuo.* Purification on silica gel afforded of ({4-[({[(Z)-(1-methyl-1H-tetrazol-5-yl)(phenyl)methylene]amino}oxy)methyl]-1,3-thiazol-2-yl}amino)({[(E)-phenylmethylene]amino}oxy)methanone as a colorless oil [132mg, yield 61% ; HPLC/MS : m/z = 463 (M+H) ; logP_{(HCOOH)} = 3.39].

### Example A

| **Phytophthora test (tomato) / preventive** | | |
|---|---|---|
| Solvent: | 49 | parts by weight of N, N - Dimethylformamide |
| Emulsifier: | 1 | part by weight of Alkylarylpolyglycolether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. One day after this treatment, the plants are inoculated with an aqueous spore suspension of *Phytophthora infestans.* The plants remain for one day in an incubation cabinet at approximately 22°C and a relative atmospheric humidity of 100%. Then the plants are placed in an incubation cabinet at approximately 20°C and a relative atmospheric humidity of 96%.

The test is evaluated 7 days after the inoculation. 0% means an efficacy which corresponds to that of the untreated control, while an efficacy of 100% means that no disease is observed.

In this test the following compounds according to the invention showed efficacy of 70% or even higher at a concentration of 100ppm of active ingredient.

| Example | Eff.% |
|---|---|
| 1 | 95 |

## Claims

1. A tetrazoyloxime derivative of formula (I) 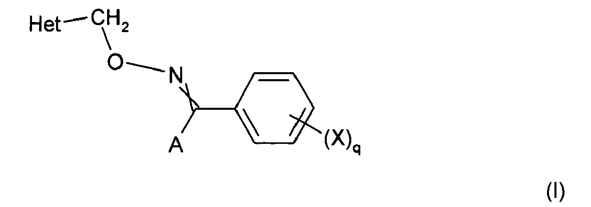 wherein
• X independently represents a hydrogen atom, a halogen atom, a nitro group, a hydroxy group, a cyano group, hydroxycarbonyl, C₁-C₈-alkoxycarbonyl, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶-sulphenyl group, a formylamino group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl-C₁-C₈-alkyl, substituted or non-substituted C₃-C₈-cycloalkyl, substituted or non-substituted tri(C₁-C₈-alkyl)silyl-C₃-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted N-(C₁-C₈-alkoxy)-C₁-C₈-alkanimidoyl, substituted or non-substituted N-(C₁-C₈-alkoxy)-C₁-C₈-halogenoalkanimidoyl having 1 to 5 halogen atoms, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms , substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted tri(C₁-C₈-alkyl)-silyl, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy, substituted or non-substituted [(arylcarbonyl)amino]-[C₁-C₈]-alkyl, substituted or non-substituted [{C₁-C₈-alkyl(C₁-C₈-alkylcarbonyl)amino}]-[C₁-C₈]-alkyl, substituted or non-substituted [{C₁-C₈-alkyl(arylcarbonyl)amino}]-[C₁-C₈]-alkyl, substituted or non-substituted [(C₁-C₈-alkylcarbonyl)amino]-[C₁-C₈]-alkyl , substituted or non-substituted heterocyclyl, substituted or non-substituted heterocyclyloxy;
• q represents 1, 2, 3, 4 or 5;
• A represents a tetrazoyl group of formula (A¹) or (A²): 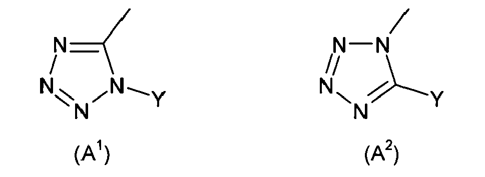 wherein Y represents substituted or non-substituted C₁-C₈-alkyl; and
• Het represents a thiazolyl group of formula: 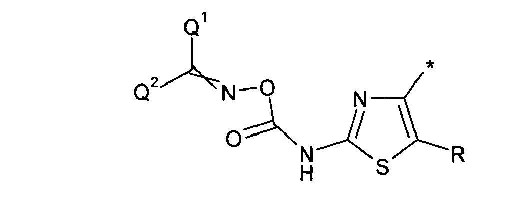 wherein
○ R represents a hydrogen atom, a halogen atom, C₁-C₈-alkyl or C₁-C₈-alkoxy and
○ Q¹ represents a hydrogen atom, a hydroxy group, a cyano group, substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted C₁-C₈-alkoxy, an amino group, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S; substituted or non-substituted C₁-C₆-alkyl-(hydroxyimino)-, substituted or non-substituted C₁-C₆-alkyl-(C₁-C₆-alkoxyimino)-, substituted or non-substituted C₁-C₆-alkyl-(C₂-C₆-alkenyloxyimino)-, substituted or non-substituted C₁-C₆-alkyl-(C₂-C₆-alkynyloxyimino)-, substituted or non-substituted C₁-C₆-alkyl-(benzyloxyimino)-, substituted or non-substituted heterocyclyl-(C₁-C₆-alkoxyimino)-, substituted or non-substituted heterocyclyl-(C₂-C₆-alkenyloxyimino)-, substituted or non-substituted heterocyclyl-(C₂-C₆-alkynyloxyimino)-, substituted or non-substituted heterocyclyl-(benzyloxyimino)-, substituted or non-substituted aryl-(C₁-C₆-alkoxyimino)-, substituted or non-substituted aryl-(C₂-C₆-alkenyloxyimino)-, substituted or non-substituted aryl-(C₂-C₆-alkynyloxyimino)-, substituted or non-substituted aryl-(benzyloxyimino)-, substituted or non-substituted C₅-C₁₂-fused bicycloalkyl, substituted or non-substituted C₅-C₁₂-fused bicycloalkenyl, substituted or non-substituted C₁-C₆-allenyl;
○ Q² represents a substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted C₁-C₈-alkoxy, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S; substituted or non-substituted C₁-C₆-alkyl-(hydroxyimino)-, substituted or non-substituted C₁-C₆-alkyl-(C₁-C₆-alkoxyimino)-, substituted or non-substituted C₁-C₆-alkyl-(C₂-C₆-alkenyloxyimino)-, substituted or non-substituted C₁-C₆-alkyl-(C₂-C₆-alkynyloxyimino)-, substituted or non-substituted C₁-C₆-alkyl-(benzyloxyimino)-, substituted or non-substituted heterocyclyl-(C₁-C₆-alkoxyimino)-, substituted or non-substituted heterocyclyl-(C₂-C₆-alkenyloxyimino)-, substituted or non-substituted heterocyclyl-(C₂-C₆-alkynyloxyimino)-, substituted or non-substituted heterocyclyl-(benzyloxyimino)-, substituted or non-substituted aryl-(C₁-C₆-alkoxyimino)-, substituted or non-substituted aryl-(C₂-C₆-alkenyloxyimino)-, substituted or non-substituted aryl-(C₂-C₆-alkynyloxyimino)-, substituted or non-substituted aryl-(benzyloxyimino)-, substituted or non-substituted C₅-C₁₂-fused bicycloalkyl, substituted or non-substituted C₅-C₁₂-fused bicycloalkenyl, substituted or non-substituted C₁-C₆-allenyl; or
o Q¹ and Q² form together a substituted or non-substituted, saturated or unsaturated 4-, 5-, 6-, 7-, 8-, 9-, 10-, or 11-membered carbocycle or heterocycle comprising up to 4 heteroatoms selected in the list consisting of N, O, S;
as well as salts, N-oxides, metallic complexes and metalloidic complexes thereof or (E) and (Z) isomers and mixtures thereof.

2. A compound according to claim 1 wherein X represents a hydrogen atom, a halogen atom, substituted or non-substituted C₁-C₈-alkyl, a substituted or non-substituted C₁-C₈-alkoxy, a cyano group, a methanesulfonyl group, a nitro group, a trifluoromethyl group or an aryl group.

3. A compound according to claim 1 or 2 wherein X represents a hydrogen atom, a chlorine atom, a fluorine atom, a methyl group, a tert-butyl group, a methoxy group, an ethoxy group, a cyano group, a methanesulfonyl group, a nitro group, a trifluoromethyl group or an aryl group.

4. A compound according to any one of claims 1 to 3 wherein X represents a hydrogen atom.

5. A compound according to any one of claims 1 to 4 wherein q represents 1.

6. A compound according to any one of claims 1 to 5 wherein Y represents a substituted or non-substituted C₁-C₈-alkyl group.

7. A compound according to any one of claims 1 to 6 wherein Y represents a methyl group or an ethyl group.

8. A compound according to any one of claims 1 to 7 wherein R represents a hydrogen atom or a halogen atom.

9. A compound according to any one of claims 1 to 8 wherein R represents a hydrogen atom or a chlorine atom

10. A compound according to any one of claims 1 to 9 wherein Q¹ represents a hydrogen atom, substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S.

11. A compound according to any one of claims 1 to 10 wherein Q¹ represents a hydrogen atom, or a substituted or non-substituted C₁-C₈-alkyl.

12. A compound according to any one of claims 1 to 11 wherein Q² represents a substituted or non-substituted C₁-C₈-alkyl, substituted or non-substituted C₃-C₈-cycloalkyl, substituted or non-substituted C₂-C₈-alkenyl, substituted or non-substituted C₂-C₈-alkynyl, substituted or non-substituted aryl, substituted or non-substituted, saturated or unsaturated 4-, 5-, 6-, 7-, 8-, 9-, 10-, or 11-membered heterocyclyl comprising up to 4 heteroatoms selected in the list consisting of N, O, S.

13. A compound according to any one of claims 1 to 12 wherein Q¹ and Q² form together a substituted or non-substituted, saturated or unsaturated 5-, 6-, 7-, 8-, 9-, 10-, or 11-membered carbocycle or heterocycle comprising up to 4 heteroatoms selected in the list consisting of N, O, S.

14. A compound according to any one of claims 1 to 13 wherein Q¹ and Q² form together a substituted or non-substituted cyclopentyl or cyclohexyl.

15. A method for controlling phytopathogenic fungi of crops, **characterized in that** an agronomically effective and substantially non-phytotoxic quantity of a compound according to claims 1 to 14 is applied to the soil where plants grow or are capable of growing, to the leaves and/or the fruit of plants or to the seeds of plants.
